# EUROPEAN PATENT APPLICATION

(11) **EP 4 442 273 A1**
(43) Date of publication of application: **09.10.2024**
(21) Application number: 22900441.1
(22) Date of filing: 29.11.2022
(51) Int. Cl.: A61K 39/395, C07K 16/46, C07K 16/36, A61P 7/02

(54) **METHOD FOR PREVENTING AND/OR TREATING THROMBOEMBOLIC DISEASES**

(30) Priority: 30.11.2021 CN 202111439560
(71) Applicant: Suzhou Alphamab Co., Ltd., Jiangsu 215125 (CN)
(72) Inventor: XU, Ting, Suzhou, Jiangsu 215125 (CN); YIN, Qun, Suzhou, Jiangsu 215125 (CN); YAN, Rongmei, Suzhou, Jiangsu 215125 (CN); LI, Qian, Suzhou, Jiangsu 215125 (CN); WANG, Yan, Suzhou, Jiangsu 215125 (CN)
(74) Representative: Weickmann & Weickmann PartmbB
(86) International application number: PCT/CN2022/134868
(87) International publication number: WO 2023/098637

(57) **Abstract**

The present invention relates to a method for preventing and/or treating thromboembolic diseases, comprising the following step: administering a coagulation factor XI (FXI) binding protein to a subject in need thereof, which binding protein can comprise at least one immunoglobulin single variable domain capable of specifically binding to FXI, wherein the at least one immunoglobulin single variable domain may comprise CDR1, CDR2, and CDR3 in a VHH as shown in any one of SEQ ID NOs: 4, 10, and 14.

## Description

### TECHNICAL FIELD

The present application relates to the field of biopharmaceuticals, and in particular, to a method for preventing and/or treating a thromboembolic disease.

### BACKGROUND

The coagulation pathway in humans includes intrinsic and extrinsic pathways. The extrinsic coagulation pathway starts from the release of TF following tissue injuries. TF forms a complex with FVII, thereby activating FIX and FX. The intrinsic coagulation pathway is prominently initiated by the activation of FXII factor binding to a negatively charged molecule, where FXII is activated to FXIIa, the activated FXIIa activates FXI to FXIa, FXIa then activates downstream FIX, and FX is activated by cofactor FVIII. FXa and FVa activated in the extrinsic and intrinsic coagulation pathways together constitute the prothrombin complex to activate prothrombin (FII), resulting in an active thrombin and fibrin clots, and ultimately resulting in coagulation.

FXI is one of the factors in the intrinsic coagulation pathway, and is present in the form of a dimer. The monomer of FXI consists of 4 Apple domains and a serine protease domain, and activated FXIa has proteolytic enzyme activity to activate its primary substrate FIX, promoting thrombin production. In addition to the substrate FIX, FXIa may also activate FX, FV, and FVIII.

The deficiency of FXI can protect against thrombotic disease. However, no drug targeting FXI is available on the market, and therefore, there is a great need for the development of antithrombotic drugs against FXI.

### SUMMARY

The present application provides a method for preventing and/or treating a thromboembolic disease. The coagulation factor XI (FXI)-binding protein of the present application can be used for effectively preventing and/or treating the thromboembolic disease, for example, for preventing thrombosis in vivo; for the effectively reducing the weight of thrombus; for prolonging APTT; and/or for reducing platelet aggregation.

In one aspect, the present application provides a method for preventing and/or treating a thromboembolic disease, comprising: administering to a subject in need a coagulation factor XI (FXI)-binding protein that may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

In some certain embodiments, the FXI-binding protein comprises a first immunoglobulin single variable domain and a second immunoglobulin single variable domain, wherein the first immunoglobulin single variable domain comprises the CDR1, CDR2, and CDR3 of the VHH set forth in SEQ ID NO: 14, and the second immunoglobulin single variable domain comprises the CDR1, CDR2, and CDR3 of the VHH set forth in SEQ ID NO: 17.

In some certain embodiments, the thromboembolic disease comprises venous thromboembolism (VTE).

In some certain embodiments, the venous thromboembolism is a complication of tumor and/or a complication of arthroplasty.

In some certain embodiments, the venous thromboembolism is associated with a peripherally inserted central catheter (PICC).

In some certain embodiments, the subject has, or is at risk of having, venous thromboembolism (VTE).

In some certain embodiments, the subject is a tumor patient.

In some certain embodiments, the subject has a peripherally inserted central catheter (PICC).

In some certain embodiments, the venous thromboembolism is associated with hip and/or knee arthroplasty.

In some certain embodiments, the subject has received arthroplasty.

In some certain embodiments, the hip and/or knee joint of the subject has been replaced.

In some certain embodiments, the thromboembolic disease includes deep vein thrombosis (DVT).

In some certain embodiments, the deep vein thrombosis includes acute deep vein thrombosis and/or recurrence of deep vein thrombosis after acute deep vein thrombosis.

In some certain embodiments, the venous thromboembolism includes pulmonary thromboembolism (PTE).

In some certain embodiments, the subject has, or is at risk of having, deep vein thrombosis (DVT).

In some certain embodiments, the subject has acute deep vein thrombosis and/or recurrence of deep vein thrombosis after acute deep vein thrombosis.

In some certain embodiments, the subject has pulmonary thromboembolism (PTE).

In some certain embodiments, the thromboembolic disease includes systemic thromboembolism.

In some certain embodiments, the systemic thromboembolism is a complication of tumor, a complication of atrial fibrillation, and/or a complication of dialysis.

In some certain embodiments, the atrial fibrillation includes non-valvular atrial fibrillation.

In some certain embodiments, the subject has, or is at risk of having, systemic thromboembolism.

In some certain embodiments, the subject has non-valvular atrial fibrillation.

In some certain embodiments, the subject is a patient receiving dialysis.

In some certain embodiments, the subject is an adult.

In some certain embodiments, the subject has hypertension, diabetes, congestive heart failure, atrial fibrillation, and/or a history of stroke.

In some certain embodiments, the subject is aged at least 75 years.

In some certain embodiments, the subject is bedridden.

In some certain embodiments, the CDRs may be Kabat CDRs, AbM CDRs, Chothia CDRs, or IMGT CDRs.

In some certain embodiments, the CDR1, CDR2, and CDR3 of the VHH set forth in SEQ ID NO: 4 are selected from any one of the following groups: SEQ ID NOs: 60-62, SEQ ID NOs: 63-65, SEQ ID NOs: 66-68, and SEQ ID NOs: 69-71.

In some certain embodiments, the at least one immunoglobulin single variable domain may comprise the amino acid sequence set forth in any one of SEQ ID NOs: 4, and 306-311.

In some certain embodiments, the CDR1, CDR2, and CDR3 of the VHH set forth in SEQ ID NO: 10 are selected from any one of the following groups: SEQ ID NOs: 132-134, SEQ ID NOs: 135-137, SEQ ID NOs: 138-140, and SEQ ID NOs: 141-143.

In some certain embodiments, the at least one immunoglobulin single variable domain may comprise the amino acid sequence set forth in any one of SEQ ID NOs: 10, and 312-317.

In some certain embodiments, the CDR1, CDR2, and CDR3 of the VHH set forth in SEQ ID NO: 14 are selected from any one of the following groups: SEQ ID NOs: 180-182, SEQ ID NOs: 183-185, SEQ ID NOs: 186-188, and SEQ ID NOs: 189-191.

In some certain embodiments, the at least one immunoglobulin single variable domain may comprise the amino acid sequence set forth in any one of SEQ ID NOs: 14, and 318-328.

In some certain embodiments, the coagulation factor XI (FXI)-binding protein binds to the Apple2 domain of FXI.

In some certain embodiments, the coagulation factor XI (FXI)-binding protein may further comprise an immunoglobulin Fc region.

In some certain embodiments, the amino acid sequence of the immunoglobulin Fc region is set forth in SEQ ID NO: 336.

In some certain embodiments, the coagulation factor XI (FXI)-binding protein is administered at a dose of about 0.5 mg/kg to about 10 mg/kg.

In some certain embodiments, the method further comprises: administering to the subject: a nucleic acid molecule encoding the coagulation factor XI (FXI)-binding protein described herein, an expression vector that may comprise the nucleic acid molecule operably linked to an expression regulatory element, a cell that may comprise the nucleic acid molecule or may be transformed with the expression vector and is capable of expressing the coagulation factor XI (FXI)-binding protein, and/or a pharmaceutical composition that may comprise the coagulation factor XI (FXI)-binding protein and a pharmaceutically acceptable carrier.

In another aspect, the present application provides use of a coagulation factor XI (FXI)-binding protein in preparing a medicament for preventing and/or treating a thromboembolic disease, wherein the coagulation factor XI (FXI)-binding protein may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

In another aspect, the present application provides a coagulation factor XI (FXI)-binding protein for use in preventing and/or treating a thromboembolic disease, wherein the coagulation factor XI (FXI)-binding protein may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

Other aspects and advantages of the present application will be readily apparent to those skilled in the art from the following detailed description. Only exemplary embodiments of the present application are shown and described in the following detailed description. As those skilled in the art will recognize, the content of the present application enables those skilled in the art to make changes to the specific embodiments disclosed without departing from the spirit and scope of the invention involved in the present application. Accordingly, descriptions in the drawings and specification are only illustrative rather than limiting.

### BRIEF DESCRIPTION OF THE DRAWINGS

Specific features of the invention involved in the present application are set forth in appended claims. Features and advantages of the invention involved in the present application will be better understood with reference to the exemplary embodiments and drawings described in detail below. A brief description of the drawings is as below.
FIG. 1 shows the prolongation effects of the coagulation factor XI (FXI)-binding proteins described in the present application on human plasma APTT.
FIG. 2 shows the prolongation effects of the coagulation factor XI (FXI)-binding proteins described in the present application on cynomolgus plasma APTT.
FIG. 3 shows the prolongation effects of the coagulation factor XI (FXI)-binding proteins described in the present application on rabbit plasma APTT.
FIG. 4 shows the prolongation effects of the coagulation factor XI (FXI)-binding proteins described in the present application on rat plasma APTT.
FIG. 5 shows the inhibitory effects of the coagulation factor XI (FXI)-binding proteins described in the present application on thrombus weight.
FIG. 6 shows the prolongation effects of the coagulation factor XI (FXI)-binding proteins described in the present application on APTT.
FIG. 7 shows the effects of the coagulation factor XI (FXI)-binding proteins described in the present application on platelet aggregation ability.
FIG. 8 shows the effects of the coagulation factor XI (FXI)-binding proteins described in the present application on PT.
FIG. 9 shows the effects of the coagulation factor XI (FXI)-binding proteins described in the present application on hemorrhage.

### DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the term "thromboembolic disease" includes diseases caused by pathological processes thrombosis and thromboembolism. Thrombosis generally refers to a pathological process in which blood constituents form emboli in blood vessels (mostly small blood vessels) in certain conditions, such that the blood vessels are partially or completely blocked, and the blood supply at corresponding parts is obstructed. Thromboembolism generally refers to a pathological process in which the thrombus falls from the formation site, partially or completely blocking some blood vessels during the process of movement with the blood flow, causing ischemia, anoxia, necrosis (arterial thrombosis) and congestion, edema (venous thrombosis) in corresponding tissues and/or organs. The thromboembolic disease may include, as per the type of vessel in which thrombosis occurs, (1) venous thromboembolic disease, i.e., venous thromboembolism, which may include, for example, pulmonary thromboembolism, deep vein thrombosis; (2) arterial thromboembolic disease, which may include, for example, acute coronary syndrome, atrial fibrillation, ischemic attacks in arteries, cerebral stroke; and (3) microvascular thrombotic disease, which may include, for example, disseminated intravascular coagulation (DIC), thrombotic thrombocytopenic purpura, and the like.

In the present application, the term "venous thromboembolism (VTE)" generally refers to a condition of intravenous blood plaque formation in which abnormal coagulation of blood in veins may cause complete or incomplete blockage of blood vessels, which is a venous reflux disorder. Venous thromboembolism may be found in deep veins of the lower limbs. Emboli may detach from the site of formation and enter the blood circulation, thus being termed embolism. The venous thromboembolism may be caused by: the interruption of blood flow (e.g., long-term bed rest, or plaster or brace immobilization, especially with comorbid dehydration and previous venous disease (chronic venous insufficiency)), damages in venous walls (e.g., surgery, trauma, or inflammation), or the trend of thrombosis (e.g., an imbalance between coagulation and fibrinolysis caused by procoagulant factors or specific drugs).

In the present application, the term "complication" generally refers to a condition where a disease causes the occurrence of an additional disease or symptom during the course of progress, the latter being the complication of the former disease. In some certain cases, the complication may also include the co-occurrence of one or more additional diseases associated with the primary disease during the treatment.

In the present application, the term "peripherally inserted central catheter (PICC)" generally refers to a catheter that is inserted into a central vein via peripheral venipuncture. The peripherally inserted central catheter can be implanted from a vein in the elbow or upper arm. It travels through the vein and is ultimately delivered to the major vessels close to the heart. The PICC is a durable, safe, and painless approach for long-term intravenous treatment and the infusion of hypertonic and irritant drugs in patients. The PICC may cause complications such as infections, thrombosis, phlebitis, etc.

In the present application, the term "arthroplasty" generally refers to a technique of manufacturing an artificial joint prosthesis according to the shape, configuration, and functionality of a joint in a human body, and surgically implanting the artificial joint prosthesis into the human body. For example, the artificial joint prosthesis may be made of artificial materials, such as metals, macromolecular polyethylene, and/or ceramics.

In the present application, the term "deep vein thrombosis (DVT)" generally refers to a condition caused by abnormal coagulation of blood in a deep vein. The deep vein thrombosis may occur in the lower limbs, and generally occurs after major orthopedic surgeries. The deep vein thrombosis may cause pulmonary embolism (PE), both of which are collectively referred to as venous thromboembolism. The deep vein thrombosis may be caused by: slow venous flow, damages to the vein walls, and hypercoagulation.

In the present application, the term "pulmonary thromboembolism (PTE)" generally refers to a disease caused by the occlusion of a pulmonary artery or its branches by thrombi from the venous system or the right heart, with pulmonary circulatory and respiratory dysfunctions being the main clinical and pathophysiological features. The PTE-causing thrombi primarily originate from deep vein thrombosis (DVT), and PTE and DVT may be collectively referred to as venous thromboembolism (VTE). The causative factors for PTE may include genetic factors (e.g., genetic variation) and acquired factors (e.g., surgery, trauma, acute medical diseases (e.g., heart failure, respiratory failure, infection, etc.), certain chronic diseases (e.g., antiphospholipid syndrome, nephrotic syndrome, etc.), and malignancies).

In the present application, the term "atrial fibrillation" is a common cardiac arrhythmia. It refers to the loss of regular and ordered atrial electrical activity and rapid and a disordered fibrillation wave replacement, and is a serious atrial electrical activity disorder. The atrial fibrillation may be associated with coronary heart disease, hypertension, and/or heart failure. The atrial fibrillation can be divided into first diagnosed atrial fibrillation, paroxysmal atrial fibrillation, persistent atrial fibrillation, long-standing persistent atrial fibrillation, and permanent atrial fibrillation. The atrial fibrillation may lead to complications such as heart failure, arterial embolism, etc., and even sudden death in severe cases.

In the present application, the term "non-valvular atrial fibrillation" refers generally to atrial fibrillation occurring in the absence of rheumatic mitral stenosis, biological or mechanical valve replacement, and mitral valve repair. In the case of non-valvular atrial fibrillation, non-vitamin K antagonist direct oral anticoagulants (DOACs) can be used to prevent stroke.

In the present application, the term "dialysis" generally refers to a separation and purification technique that separates micromolecules from biological macromolecules. It refers to an artificial process for clearing metabolic wastes and excessive water from the body when the kidneys cannot work normally. The dialysis (therapy) may include a treatment method in which components (solutes or moisture) in the body fluids are removed from the body through a semi-permeable membrane, and may generally include hemodialysis, peritoneal dialysis, and/or colonic dialysis.

In the present application, the term "congestive heart failure (CHF)" generally refers to hypoperfusion in tissues and/or organs with the co-occurrence of congestion in pulmonary and/or systemic circulation, which is a clinical syndrome when various heart diseases progress to a severe stage. The congestive heart failure may be caused by the unsatisfied metabolic need for blood due to ventricular pumping or filling dysfunctionality. The congestive heart failure may be characterized by left ventricular hypertrophy or dilatation and may lead to neuroendocrine disorders and circulatory dysfunction and to typical clinical symptoms such as dyspnea, body fluid retention, and asthenia.

In the present application, the term "hypertension" generally refers to a clinical syndrome characterized by an increase in systemic arterial blood pressure (systolic pressure and/or diastolic pressure; in the case where a hypotensive drug is not used, a systolic pressure of 140 mmHg or higher and/or a diastolic pressure of 90 mmHg or higher), which may be accompanied by functional or organic damages to organs such as heart, brain, kidney, etc. The hypertension can be the prominent risk factor for cardiovascular and cerebrovascular diseases.

In the present application, the term "diabetes" generally refers to a group of metabolic diseases characterized by chronic hyperglycemia caused by disorders of insulin secretion and/or utilization due to multiple causes. The long-term carbohydrate, fat, and protein metabolism disorder may cause multi-system dysfunction, and lead to chronic progressive pathological changes, hypofunctionality, and failure of tissues and organs such as eyes, kidneys, nerves, heart, blood vessels, and the like. The diabetes may include type 1, type 2, other specific types, and gestational diabetes.

In the present application, the term "bedridden" generally refers to a clinical condition in which the daily life ability is reduced due to long-term illness or injuries and partial or complete assistance is required.

Unless otherwise indicated, the terms "antibody" and "immunoglobulin" used interchangeably herein, whether referring to a heavy-chain antibody or to a conventional 4-chain antibody, are used as a general term to include full-length antibodies, individual chains thereof, and all portions, domains or fragments thereof (including, but not limited to, antigen-binding domains or fragments, such as VHH domains or VH/VL domains, respectively). In addition, the term "sequence" used herein (e.g., in terms "immunoglobulin sequence", "antibody sequence", "single variable domain sequence", "VHH sequence", or "protein sequence") is generally intended to encompass related amino acid sequences and nucleic acid or nucleotide sequences encoding the sequences, unless further limited interpretation is required herein.

The term "domain" as used herein (of a polypeptide or protein) refers to a folded protein structure that is capable of maintaining its tertiary structure independently of the rest of the protein. In general, a domain is responsible for a single functional property of a protein, and in many cases may be added, removed, or transferred to other proteins without compromising the functionality of the rest of the protein and/or the domain.

The term "immunoglobulin domain" as used herein refers to a globular region of an antibody chain (e.g., a chain of a conventional 4-chain antibody or a chain of a heavy chain antibody) or a polypeptide essentially consisting of such globular regions. The immunoglobulin domain is characterized by retaining the immunoglobulin folding characteristics of an antibody molecule.

The term "immunoglobulin variable domain" as used herein refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are spaced apart by three "complementarity determining regions" or "CDRs" referred to in the art and hereinafter as "complementarity determining region 1" or "CDR1", "complementarity determining region 2" or "CDR2", and "complementarity determining region 3" or "CDR3". Thus, the general structure or sequence of an immunoglobulin variable domain can be represented as follows: FR1-CDR1-FR2-CDR2-FR3-CDR3-FR4. The immunoglobulin variable domain endows the antibody with specificity for antigens due to the antigen-binding site.

The term "immunoglobulin single variable domain" as used herein refers to an immunoglobulin variable domain that is capable of specifically binding to an epitope of an antigen without pairing with other immunoglobulin variable domains. An example of the immunoglobulin single variable domain in the context of the present application is the "domain antibody", such as immunoglobulin single variable domains VH and VL (VH domain and VL domain). Another example of the immunoglobulin single variable domain is the "VHH domain" (or abbreviated as "VHH") of the family Camelidae as defined below.

The "VHH domain", also known as heavy chain single-domain antibody, VHH, VHH domain, VHH antibody fragment and VHH antibody, is the variable domain of an antigen-binding immunoglobulin known as the "heavy chain antibody" (i.e., an "antibody devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" is used to distinguish the variable domain from the heavy chain variable domain (which is referred to herein as a "VH domain") present in a conventional 4-chain antibody and the light chain variable domain (which is referred to herein as a "VL domain") present in a conventional 4-chain antibody. The VHH domain specifically binds to an epitope without the need for additional antigen-binding domains (as opposed to the VH or VL domain in a conventional 4-chain antibody, in which the epitope is recognized by the cooperation of the VL domain and the VH domain). The VHH domain is a small, stable, and efficient antigen recognition unit formed by a single immunoglobulin domain.

In the context of the present application, the terms "heavy chain single-domain antibody", "VHH domain", "VHH", "VHH domain", "VHH antibody fragment", and "VHH antibody" can be used interchangeably.

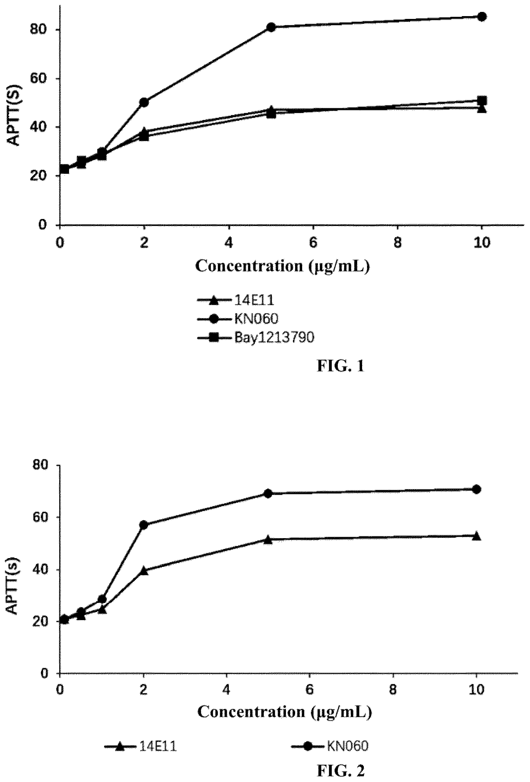

For example, as shown in FIG. 2 in Riechmann and Muyldermans, J.Immunol. Methods 231, 25-38 (1999), the amino acid residues used in VHH domains of the family Camelidae may be numbered according to the general numbering method of VH domains given by Kabat et al. (Kabat et al., Sequences of Proteins of Immunological Interest, 5th Ed. Public Health Service, National Institutes of Health, Bethesda, Md. (1991)).

Alternative methods for numbering amino acid residues of VH domains are known in the art and may also be similarly applied to VHH domains. For example, the Chothia CDRs are based on the positions of structural loops (Chothia and Lesk, J. Mol. Biol. 196:901-917 (1987)). The AbM CDRs are a compromise based on the Kabat hypervariable regions and Chothia structure loops, and are used in Oxford Molecular's AbM antibody modeling software. The "Contact" CDRs are based on the analysis of available complex crystal structures. The residues of CDRs from the methods are described below:

| Loop | Kabat | AbM | Chothia | Contact |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 (Kabat numbering) | H31-H35B | H26-H35B | H26-H32 | H30-H35B |
| HCDR1 (Chothia numbering) | H31-H35 | H26-H35 | H26-H32 | H30-H35 |
| HCDR2 | H50-H65 | H50-H58 | H53-H55 | H47-H58 |
| HCDR3 | H95-H102 | H95-H102 | H96-H101 | H93-H101 |

However, it should be noted that, as is well known in the art for VH domains and for VHH domains, the total number of amino acid residues in each CDR may vary and may not correspond to the total number of amino acid residues indicated by the Kabat numbering (that is, one or more positions according to the Kabat numbering may not be occupied in the actual sequence, or the actual sequence may contain more amino acid residues than the number permitted by the Kabat numbering). This means that, generally, the numbering according to Kabat may or may not correspond to the actual numbering of the amino acid residues in actual sequences.

For example, CDRs may include "extended CDRs", such as 24-36 or 24-34 (LCDR1), 46-56 or 50-56 (LCDR2), and 89-97 or 89-96 (LCDR3) in the VL; 26-35 (HCDR1), 50-65 or 49-65 (HCDR2), and 93-102, 94-102 or 95-102 (HCDR3) in the VH.

The total number of amino acid residues in a VHH domain will usually be in the range of 110 to 120, often between 112 and 115. However, it should be noted that shorter and longer sequences may also be suitable for the purposes described herein.

Other structural and functional properties of VHH domains and polypeptides containing the same are summarized as follows:

the VHH domain (which has been naturally "designed" to functionally bind to an antigen without the presence of or interaction with a light chain variable domain) can be used as a single and relatively-small functional antigen-binding structural unit, domain, or polypeptide. This distinguishes VHH domains from the VH and VL domains of conventional 4-chain antibodies, wherein the VH and VL domains per se are generally not suitable for practical application as single antigen binding proteins or immunoglobulin single variable domains, but need to be combined in a certain form or another form to provide functional antigen-binding units (e.g., in the form of a conventional antibody fragment such as a Fab fragment; or in the form of an scFv consisting of a VH domain covalently linked to a VL domain).

Due to these unique properties, compared with conventional VH and VL domains, scFv and conventional antibody fragments (e.g., Fab- or F(ab')₂ fragments), VHH domains, either alone or as part of a larger polypeptide, offer a number of superior significant advantages: only a single domain is required to bind to an antigen with high affinity and high selectivity, such that there is no need to retain two separate domains, nor to assure that these two domains are present in the proper spatial conformation and configuration (e.g., the use of specially designed linker is generally required for an scFv); VHH domains can be expressed from a single gene and do not require post-translational folding or modification; VHH domains can be easily engineered into multivalent and multi-specific formats (formatting); VHH domains are highly soluble and do not have a tendency to aggregate; VHH domains are highly stable to heat, pH, proteases and other denaturing agents or conditions and thus, may be prepared, stored, or transported without the use of refrigeration equipment, thereby possessing cost-efficiency, efficiency, and environment-friendliness; VHH domains are easy to prepare and relatively inexpensive, even on a manufacturing scale; VHH domains are relatively small compared with conventional 4-chain antibodies and antigen-binding fragments thereof (about 15 kDa or 1/10 of conventional IgG in size), and therefore exhibit higher tissue permeability and can be administered at higher doses compared with conventional 4-chain antibodies and antigen-binding fragments thereof; VHH domains may exhibit so-called cavity-binding properties (particularly due to their extended CDR3 loop, compared with conventional VH domains) and can therefore reach targets and epitopes not available for conventional 4-chain antibodies and antigen-binding fragments thereof.

Methods for obtaining VHHs that bind to a particular antigen or epitope have been previously disclosed in the following documents: R. van der Linden et al., Journal of Immunological Methods, 240(2000)185-195; Li et al., J Biol Chem., 287(2012)13713-13721; Deffar et al., African Journal of Biotechnology Vol. 8(12), pp. 2645-2652, 17 June, 2009; and WO94/04678.

The "humanization" of VHH domains derived from the family Camelidae can be obtained by replacing one or more amino acid residues in the amino acid sequence of the original VHH sequence with one or more amino acid residues present at the corresponding positions in a VH domain of a human conventional 4-chain antibody (also referred to herein as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. Humanization can be accomplished using methods for protein surface amino acid humanization (resurfacing) and/or humanized CDR grafting to a universal framework, for example, as exemplified in the examples.

As used herein, the term "epitope" or "antigenic determinant" used interchangeably herein refers to any antigenic determinant on an antigen to which the paratope of an antibody binds. The antigenic determinant generally comprises chemically active surface groups of molecules such as amino acids or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be a "linear" epitope or a "conformational" epitope. See, e.g., Epitope Mapping Protocols in Methods in Molecular Biology, vol. 66, G. E. Morris, Ed. (1996). In a linear epitope, all points of interaction between a protein and an interacting molecule (e.g., an antibody) are present linearly along the primary amino acid sequence of the protein. In a conformational epitope, the points of interaction are present across amino acid residues on the protein that are separated from one another.

Epitopes of a given antigen can be identified using a number of epitope mapping techniques well known in the art. See, e.g., Epitope Mapping Protocols in Methods in Molecular Biology, vol. 66, G. E. Morris, Ed. (1996). For example, a linear epitope can be determined by, for example, the method as follows: a plurality of peptides corresponding to portions of the protein molecule are synthesized simultaneously on a solid support, and these peptides are reacted with the antibody while still attached to the support. Such techniques are known in the art and are described, for example, in U.S. Pat. Nos. 4,708,871; Geysen et al. (1984) Proc. Natl. Acad. Sci. USA 81:3998-4002; Geysen et al. (1986) Molec. Immunol. 23:709-715. Similarly, conformational epitopes can be identified by determining the spatial configuration of amino acids, for example, by X-ray crystallography and two-dimensional nuclear magnetic resonance. See, e.g., Epitope Mapping Protocols (supra).

Antibodies can be screened for competitively binding to the same epitope using conventional techniques known to those skilled in the art. For example, competition and cross-competition studies can be performed to give antibodies that compete or cross-compete with one another for binding to an antigen. A high-throughput method for acquiring antibodies that bind to the same epitope based on their cross-competition is described in International Patent Application No. WO03/48731. Therefore, an antibody and an antigen-binding fragment thereof that competes with the antibody molecule of the present application for binding to the same epitope on FXI can be obtained by conventional techniques known to those skilled in the art.

In general, the term "specificity" refers to the number of different types of antigens or epitopes to which a particular antigen-binding molecule or antigen-binding protein (e.g., an immunoglobulin single variable domain of the present application) can bind. The specificity of an antigen-binding protein can be determined based on its affinity and/or avidity. Affinity, represented by a dissociation equilibrium constant (KD) of an antigen to an antigen-binding protein, is a measure of the binding strength between an epitope and an antigen-binding site on the antigen-binding protein: a smaller KD value indicates a stronger binding strength between the epitope and the antigen-binding protein (or, the affinity can also be expressed as an association constant (KA), which is 1/KD). As will be appreciated by those skilled in the art, affinity can be determined in a known manner depending on the particular antigen of interest. Avidity is a measure of the binding strength between an antigen-binding protein (e.g., an immunoglobulin, an antibody, an immunoglobulin single variable domain, or a polypeptide containing the same) and a related antigen. Avidity is related to both the affinity for its antigen-binding site on the antigen-binding protein and the number of related binding sites present on the antigen-binding protein.

The term "coagulation factor XI (FXI)-binding protein" as used herein refers to any protein capable of specifically binding to coagulation factor XI (FXI). The FXI-binding protein may include antibodies as defined herein against FXI. The FXI-binding protein also encompasses immunoglobulin superfamily antibodies (IgSF) or CDR-grafted molecules.

The "FXI-binding protein" of the present application may comprise at least one immunoglobulin single variable domain (e.g., VHH) that binds to FXI. In the present application, the "FXI-binding molecule" of the present application may comprise 2, 3, 4, or more immunoglobulin single variable domains, such as VHHs, that bind to FXI. The FXI-binding protein of the present application may also comprise, in addition to the immunoglobulin single variable domain binding to FXI, a linker and/or a moiety with effector functions, such as a half-life extending moiety (e.g., an immunoglobulin single variable domain that binds to serum albumin), and/or a fusion partner (e.g., serum albumin) and/or a conjugated polymer (e.g., PEG) and/or an Fc region. In the present application, the "FXI-binding protein" of the present application also encompasses bispecific antibodies, which contain immunoglobulin single variable domains that bind to different antigens or different regions (e.g., different epitopes) of the same antigen.

Typically, the FXI-binding protein of the present application will bind to the antigen of interest (i.e., FXI) with a dissociation constant (KD) from 10⁻⁷ to 10⁻¹⁰ moles/liter (M), from 10⁻⁸ to 10⁻¹⁰ moles/liter, and even 10⁻⁹ to 10⁻¹⁰ or less, as measured in a Biacore or KinExA or Fortibio assay, and/or with an association constant (KA) of at least 10⁷ M⁻¹, at least 10⁸ M⁻¹, at least 10⁹ M⁻¹, or at least 10¹⁰ M⁻¹. Any KD value greater than 10⁻⁴ M is generally considered to indicate a non-specific binding. Specific binding of an antigen-binding protein to an antigen or epitope can be determined in any known suitable way, including, for example, surface plasmon resonance (SPR) assay, Scatchard assay, and/or competitive binding assay (e.g., radioimmunoassay (RIA), enzyme immunoassay (EIA), and sandwich competitive assay) described herein.

The "sequence identity" between two polypeptide sequences indicates the percentage of identical amino acids between the sequences. The "sequence similarity" indicates the percentage of amino acids that are identical or represent conservative amino acid substitutions. Methods for assessing the degree of sequence identity between amino acids or nucleotides are known to those skilled in the art. For example, amino acid sequence identity is typically measured using sequence analysis software. For example, the BLAST program from the NCBI database can be used to determine the identity. For the determination of sequence identity, see, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987 and Sequence Analysis Primer, Gribskov, M. and Devereux, J., eds., M Stockton Press, New York, 1991.

Compared with the natural biological source of a polypeptide or nucleic acid molecule and/or the reaction medium or culture medium used for acquiring the polypeptide or nucleic acid molecule, when the polypeptide or nucleic acid molecule has been separated from at least one other usually associated component (such as another protein/polypeptide, another nucleic acid, another biological component or macromolecule or at least one pollutant, impurity, or trace component) in the source or medium (culture medium), the polypeptide or nucleic acid molecule is regarded as "isolated". In particular, a polypeptide or nucleic acid molecule is considered "isolated" when it has been purified at least 2-fold, in particular at least 10-fold, more in particular at least 100-fold and up to 1000-fold or more than 1000-fold. The "isolated" polypeptide or nucleic acid molecule is preferably substantially homogeneous, as determined by suitable techniques (e.g., suitable chromatographic techniques, such as polyacrylamide gel electrophoresis).

The "effective amount" refers to the amount of the FXI-binding protein or the pharmaceutical composition of the present application that results in a reduction in the severity of disease symptoms, an increase in the frequency and duration of asymptomatic period of the disease, or the prevention of damages or disability resulting from the affliction of the disease.

As used herein, "thrombosis" refers to the formation or presence of a clot (also referred to as "thrombus") within a blood vessel, thereby impeding the flow of blood through the circulatory system. Thrombosis is typically caused by abnormalities in the composition of the blood, the quality of the vessel wall, and/or the nature of the blood flow. The formation of a clot is typically caused by damages to the vessel walls (such as damages to the vessel walls due to trauma or infection) and the slowing or cessation of blood flow through the damaged site. In some cases, coagulation abnormalities cause thrombosis.

### Detailed Description of the Invention

In one aspect, the present application provides a method for preventing and/or treating a thromboembolic disease, comprising: administering to a subject in need a coagulation factor XI (FXI)-binding protein that may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

In another aspect, the present application provides use of a coagulation factor XI (FXI)-binding protein in preparing a medicament for preventing and/or treating a thromboembolic disease, wherein the coagulation factor XI (FXI)-binding protein may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

In another aspect, the present application provides a coagulation factor XI (FXI)-binding protein for use in preventing and/or treating a thromboembolic disease, wherein the coagulation factor XI (FXI)-binding protein may comprise at least one immunoglobulin single variable domain capable of specifically binding to FXI, wherein the at least one immunoglobulin single variable domain may comprise the CDR1, CDR2, and CDR3 of the VHH set forth in any one of SEQ ID NOs: 4, 10, and 14.

For example, the FXI-binding protein comprises a first immunoglobulin single variable domain and a second immunoglobulin single variable domain, wherein the first immunoglobulin single variable domain comprises the CDR1, CDR2, and CDR3 of the VHH set forth in SEQ ID NO: 14, and the second immunoglobulin single variable domain comprises the CDR1, CDR2, and CDR3 of the VHH set forth in SEQ ID NO: 17.

In the present application, the method may further comprise: administering to the subject: a nucleic acid molecule encoding the coagulation factor XI (FXI)-binding protein described herein, an expression vector that may comprise the nucleic acid molecule operably linked to an expression regulatory element, a cell that may comprise the nucleic acid molecule or may be transformed with the expression vector and is capable of expressing the coagulation factor XI (FXI)-binding protein, and/or a pharmaceutical composition that may comprise the coagulation factor XI (FXI)-binding protein and a pharmaceutically acceptable carrier.

In the present application, the thromboembolic disease may include venous thromboembolism (VTE).

For example, the venous thromboembolism may be a complication of tumor and/or a complication of arthroplasty.

For example, the venous thromboembolism may be associated with a peripherally inserted central catheter (PICC).

For example, the venous thromboembolism may be associated with hip and/or knee arthroplasty. In an arthroplasty, the lower limb muscle contraction activity may be reduced, and the bleeding at the incision of the replacement surgery may make the blood more prone to clotting, possibly resulting in deep vein thrombosis in the lower limb.

For example, the thromboembolic disease may include deep vein thrombosis (DVT).

For example, the deep vein thrombosis may include acute deep vein thrombosis and/or recurrence of deep vein thrombosis after acute deep vein thrombosis.

For example, the venous thromboembolism may include pulmonary thromboembolism (PTE). For example, a deep vein thrombus may return to the lungs along with the blood, thereby blocking a non-major blood vessel. The pulmonary thromboembolism may lead to fatal pulmonary embolism.

For example, the thromboembolic disease may include systemic thromboembolism. For example, the systemic thromboembolism may be a complication of tumor, a complication of atrial fibrillation, and/or a complication of dialysis. In the present application, the atrial fibrillation may include non-valvular atrial fibrillation.

In the present application, the subject may have, or be at risk of having, venous thromboembolism (VTE).

In the present application, the subject may have a peripherally inserted central catheter (PICC).

In the present application, the subject may have received arthroplasty. For example, the hip and/or knee joint of the subject may have been replaced.

In the present application, the subject may have, or be at risk of having, deep vein thrombosis (DVT).

In the present application, the subject may have acute deep vein thrombosis and/or recurrence of deep vein thrombosis after acute deep vein thrombosis.

In the present application, the subject may have pulmonary thromboembolism (PTE).

In the present application, the subject may have, or be at risk of having, systemic thromboembolism.

In the present application, the subject may have non-valvular atrial fibrillation.

In the present application, the subject may be a patient receiving dialysis. The patient receiving dialysis may have undergone and/or is undergoing hemodialysis. The hemodialysis may include procedures of removing various harmful and unwanted metabolic wastes and excessive electrolytes in the blood out of the body by diffusion via a semi-permeable membrane. The hemodialysis can achieve the purposes of purifying blood and correcting the imbalance of electrolytes and acids/bases. The patient receiving dialysis may have undergone and/or is undergoing peritoneal dialysis. The peritoneal dialysis may include procedures of using the peritoneum as a semi-permeable membrane, periodically filling a prepared dialysate into the peritoneal cavity of a patient via a catheter by means of gravity, and continuously replacing the peritoneal dialysate. The peritoneal dialysis can achieve the purposes of removing in-vivo metabolites and toxic substances and correcting the imbalance of water and electrolytes. In the present application, the patient receiving dialysis may develop cardio-cerebral complications. For example, symptoms of hypertension, cerebral hemorrhage, and/or heart failure may occur or already exist during the dialysis.

In the present application, the subject may be an adult. In the present application, the adult may be aged eighteen years or older.

In the present application, the subject may be a tumor patient. In the present application, the tumor may be a malignant tumor. In the present application, the tumor may include solid tumors and/or non-solid tumors.

In the present application, the subject may have hypertension, diabetes, congestive heart failure, atrial fibrillation, and/or a history of stroke. In the present application, the stroke may include cerebral stroke. The cerebral stroke may be caused by the following causes: carotid arterial stenosis, atrial fibrillation, cerebral hemorrhage, and/or ischemic cerebral stroke. For the treatment of the cerebral stroke, please refer to the Chinese Series Guidelines for Stroke (2015 Edition).

In the present application, the subject may be aged at least 75 years (e.g., may be aged at least 75 years, at least 80 years, at least 85 years, or older).

In the present application, the subject may be bedridden (e.g., may be on bed rest for at least 3 months, at least 6 months, at least 1 year, at least 2 years, at least 5 years, or longer).

In the present application, the coagulation factor XI (FXI)-binding protein may be administered at a dose of about 0.5 mg/kg to about 10 mg/kg, for example, about 0.5 mg/kg to about 8.0 mg/kg, about 0.5 mg/kg to about 7.5 mg/kg, about 0.5 mg/kg to about 7.0 mg/kg, about 0.5 mg/kg to about 6.5 mg/kg, about 0.5 mg/kg to about 6 mg/kg, about 0.5 mg/kg to about 5.5 mg/kg, about 0.5 mg/kg to about 5.0 mg/kg, about 0.5 mg/kg to about 4.5 mg/kg, about 0.5 mg/kg to about 4.0 mg/kg, about 0.5 mg/kg to about 3.5 mg/kg, about 0.5 mg/kg to about 3.0 mg/kg, about 0.5 mg/kg to about 2.5 mg/kg, about 0.5 mg/kg to about 2.0 mg/kg, about 0.5 mg/kg to about 1.5 mg/kg, about 0.5 mg/kg to about 1.0 mg/kg, about 1.0 mg/kg to about 10 mg/kg, or about 1.0 mg/kg to about 8.0 mg/kg.

### FXI-binding protein

In one aspect, the present application provides an FXI-binding protein that may comprise at least one immunoglobulin single variable domain capable of specifically binding to FXI.

In the present application, the at least one immunoglobulin single variable domain may comprise the CDR1, CDR2, and CDR3 of the VHH set forth in any one of SEQ ID NOs: 1-23. The CDRs may be Kabat CDRs, AbM CDRs, Chothia CDRs, or IMGT CDRs.

In the present application, the at least one immunoglobulin single variable domain may comprise a set of CDR1, CDR2, and CDR3 selected from the following:

| Antibody strain No. | CDR1-3 according to Kabat | CDR1-3 according to AbM | CDR1-3 according to Chothia | CDR1-3 according to IMGT |
|---|---|---|---|---|
| 5 | SEQ ID NO:24-26 | SEQ ID NO:27-29 | SEQ ID NO:30-32 | SEQ ID NO:33-35 |
| 7 | SEQ ID NO:36-38 | SEQ ID NO:39-41 | SEQ ID NO:42-44 | SEQ ID NO:45-47 |
| 11 | SEQ ID NO:48-50 | SEQ ID NO:51-53 | SEQ ID NO:54-56 | SEQ ID NO:57-59 |
| 13 | SEQ ID NO:60-62 | SEQ ID NO:63-65 | SEQ ID NO:66-68 | SEQ ID NO:69-71 |
| 15 | SEQ ID NO:72-74 | SEQ ID NO:75-77 | SEQ ID NO:78-80 | SEQ ID NO:81-83 |
| 17 | SEQ ID NO:84-86 | SEQ ID NO:87-89 | SEQ ID NO:90-92 | SEQ ID NO:93-95 |
| 22 | SEQ ID NO:96-98 | SEQ ID NO:99-101 | SEQ ID NO:102-104 | SEQ ID NO:105-107 |
| 29 | SEQ ID NO:108-110 | SEQ ID NO:111-113 | SEQ ID NO:114-116 | SEQ ID NO:117-119 |
| 30 | SEQ ID NO:120-122 | SEQ ID NO:123-125 | SEQ ID NO:126-128 | SEQ ID NO:129-131 |
| 35 | SEQ ID NO:132-134 | SEQ ID NO:135-137 | SEQ ID NO:138-140 | SEQ ID NO:141-143 |
| 43 | SEQ ID NO:144-146 | SEQ ID NO:147-149 | SEQ ID NO:150-152 | SEQ ID NO:153-155 |
| 49 | SEQ ID NO:156-158 | SEQ ID NO:159-161 | SEQ ID NO:162-164 | SEQ ID NO:165-167 |
| 50 | SEQ ID NO:168-170 | SEQ ID NO:171-173 | SEQ ID NO:174-176 | SEQ ID NO:177-179 |
| 56 | SEQ ID NO:180-182 | SEQ ID NO:183-185 | SEQ ID NO:186-188 | SEQ ID NO:189-191 |
| 70 | SEQ ID NO:192-194 | SEQ ID NO:195-197 | SEQ ID NO:198-200 | SEQ ID NO:201-203 |
| 96 | SEQ ID N0:204-206 | SEQ ID NO:207-209 | SEQ ID NO:210-212 | SEQ ID NO:213-215 |
| 97 | SEQ ID NO:216-218 | SEQ ID NO:219-221 | SEQ ID NO:222-224 | SEQ ID NO:225-227 |
| 107 | SEQ ID NO:228-230 | SEQ ID NO:231-233 | SEQ ID NO:234-236 | SEQ ID NO:237-239 |
| 128 | SEQ ID NO:240-242 | SEQ ID NO:243-245 | SEQ ID NO:246-248 | SEQ ID NO:249-251 |
| 148 | SEQ ID NO:252-254 | SEQ ID NO:255-257 | SEQ ID NO:258-260 | SEQ ID NO:261-263 |
| 163 | SEQ ID NO:264-266 | SEQ ID NO:267-269 | SEQ ID NO:270-272 | SEQ ID NO:273-275 |
| 166 | SEQ ID NO:276-278 | SEQ ID NO:279-281 | SEQ ID NO:282-284 | SEQ ID NO:285-287 |
| 168 | SEQ ID NO:288-290 | SEQ ID NO:291-293 | SEQ ID NO:294-296 | SEQ ID NO:297-299 |

In the present application, at least one immunoglobulin single variable domain in the FXI-binding protein of the present application is a VHH. In the present application, the VHH may comprise any amino acid sequence set forth in SEQ ID NOs: 1-23.

In the present application, at least one immunoglobulin single variable domain in the FXI-binding protein of the present application is a humanized VHH.

In the present application, at least one immunoglobulin single variable domain in the FXI-binding protein of the present application is a humanized VHH that may comprise an amino acid sequence having at least 80%, feasibly at least 90%, feasibly at least 95%, or even feasibly at least 99% sequence identity to any sequence set forth in SEQ ID NOs: 1-23. In the present application, the amino acid sequence of the humanized VHH may comprise one or more amino acid substitutions, which may be conservative amino acid substitutions, compared to any one of SEQ ID NOs: 1 to 23, for example, 1, 2, 3, 4, 5, 6, 7, 8, 9, or 10 conservative amino acid substitutions.

In the present application, at least one immunoglobulin single variable domain in the FXI-binding protein of the present application is a humanized VHH comprising any amino acid sequence set forth in SEQ ID NOs: 300-335.

In the present application, the at least one immunoglobulin single variable domain binds to the Apple2 domain of FXI. For example, the immunoglobulin single variable domain may comprise the CDR1, CDR2, and CDR3 of the VHH set forth in any one of SEQ ID NO: 4, SEQ ID NO: 10, or SEQ ID NO: 14. In the present application, the immunoglobulin single variable domain may comprise a set of CDR1, CDR2, and CDR3 selected from SEQ ID NOs: 60-62, SEQ ID NOs: 63-65, SEQ ID NOs: 66-68, SEQ ID NOs: 69-71, SEQ ID NOs: 132-134, SEQ ID NOs: 135-137, SEQ ID NOs: 138-140, SEQ ID NOs: 141-143, SEQ ID NOs: 180-182, SEQ ID NOs: 183-185, SEQ ID NOs: 186-188, and SEQ ID NOs: 189-191. In the present application, the immunoglobulin single variable domain may comprise the amino acid sequence set forth in any one of SEQ ID NO: 4, SEQ ID NO: 10, or SEQ ID NO: 14. In the present application, the immunoglobulin single variable domain may comprise the amino acid sequence set forth in any one of SEQ ID NOs: 306-323.

In the present application, the at least one immunoglobulin single variable domain binds to the Apple3 domain of FXI. For example, the immunoglobulin single variable domain may comprise the CDR1, CDR2, and CDR3 of the VHH set forth in SEQ ID NO: 17. In the present application, the immunoglobulin single variable domain may comprise a set of CDR1, CDR2, and CDR3 selected from SEQ ID NOs: 216-218, SEQ ID NOs: 219-221, SEQ ID NOs: 222-224, and SEQ ID NOs: 225-227. In the present application, the immunoglobulin single variable domain may comprise the amino acid sequence set forth in SEQ ID NO: 17. In the present application, the immunoglobulin single variable domain may comprise the amino acid sequence set forth in any one of SEQ ID NOs: 324-329.

In the present application, the at least one immunoglobulin single variable domain binds to the Apple4 domain of FXI. For example, the immunoglobulin single variable domain may comprise the CDR1, CDR2, and CDR3 of the VHH set forth in SEQ ID NO: 1. In the present application, the immunoglobulin single variable domain may comprise a set of CDR1, CDR2, and CDR3 selected from SEQ ID NOs: 24-26, SEQ ID NOs: 27-29, SEQ ID NOs: 30-32, and SEQ ID NOs: 33-35. In the present application, the immunoglobulin single variable domain may comprise the amino acid sequence set forth in SEQ ID NO: 1. In the present application, the immunoglobulin single variable domain may comprise the amino acid sequence set forth in any one of SEQ ID NOs: 300-305.

In the present application, the at least one immunoglobulin single variable domain binds to the Apple1-2 region of FXI (a region between the Apple1 domain and the Apple2 domain).

In the present application, the at least one immunoglobulin single variable domain binds to the Apple2-3 region of FXI (a region between the Apple2 domain and the Apple3 domain). For example, the immunoglobulin single variable domain may comprise the CDR1, CDR2, and CDR3 of the VHH set forth in SEQ ID NO: 20. In the present application, the immunoglobulin single variable domain may comprise a set of CDR1, CDR2, and CDR3 selected from SEQ ID NOs: 252-254, SEQ ID NOs: 255-257, SEQ ID NOs: 258-260, and SEQ ID NOs: 261-263. In the present application, the immunoglobulin single variable domain may comprise the amino acid sequence set forth in SEQ ID NO: 20. In the present application, the immunoglobulin single variable domain may comprise the amino acid sequence set forth in any one of SEQ ID NOs: 330-335.

In the present application, the at least one immunoglobulin single variable domain binds to the Apple3-4 region of FXI (a region between the Apple3 domain and the Apple4 domain).

In the present application, the FXI-binding protein may comprise an immunoglobulin single variable domain that specifically binds to FXI.

In the present application, the FXI-binding protein may comprise at least two, e.g., 2, 3, 4, or more immunoglobulin single variable domains that specifically bind to FXI.

In the present application, the at least two immunoglobulin single variable domains bind to, or compete for binding to or partially compete for binding to, the same region or epitope of FXI, e.g., the at least two immunoglobulin single variable domains are identical.

In the present application, the at least two immunoglobulin single variable domains bind to different regions or epitopes of FXI, or do not compete for binding to the same region or epitope of FXI.

Whether two antibodies or immunoglobulin single variable domains bind to, or compete for binding to, the same region or epitope can be determined through epitope binning by the bio-layer interferometry (BLI), as exemplified in the examples herein.

In the present application, the at least two immunoglobulin single variable domains that specifically bind to FXI are directly linked to each other.

In the present application, the at least two immunoglobulin single variable domains that specifically bind to FXI are linked to each other via a linker. The linker may be a non-functional amino acid sequence having 1-20 or more amino acids in length and no secondary or higher-order structure. For example, the linker is a flexible linker, such as GGGGS, GS, GAP, and (GGGGS)₃.

In the present application, the FXI-binding protein may comprise a first immunoglobulin single variable domain and a second immunoglobulin single variable domain, wherein
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 4; or
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 9; or
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 10; or
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 14; or
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 17; or
the first immunoglobulin single variable domain may comprise the CDR1, CDR2, and CDR3 of the VHH set forth in SEQ ID NO: 1, and the second immunoglobulin single variable domain may comprise the CDR1, CDR2, and CDR3 of the VHH set forth in SEQ ID NO: 20; or
the first immunoglobulin single variable domain may comprise the CDR1, CDR2, and CDR3 of the VHH set forth in SEQ ID NO: 4, and the second immunoglobulin single variable domain may comprise the CDR1, CDR2, and CDR3 of the VHH set forth in SEQ ID NO: 9; or
the first immunoglobulin single variable domain may comprise the CDR1, CDR2, and CDR3 of the VHH set forth in SEQ ID NO: 4, and the second immunoglobulin single variable domain may comprise the CDR1, CDR2, and CDR3 of the VHH set forth in SEQ ID NO: 10; or
the first immunoglobulin single variable domain may comprise the CDR1, CDR2, and CDR3 of the VHH set forth in SEQ ID NO: 4, and the second immunoglobulin single variable domain may comprise the CDR1, CDR2, and CDR3 of the VHH set forth in SEQ ID NO: 14; or
the first immunoglobulin single variable domain may comprise the CDR1, CDR2, and CDR3 of the VHH set forth in SEQ ID NO: 4, and the second immunoglobulin single variable domain may comprise the CDR1, CDR2, and CDR3 of the VHH set forth in SEQ ID NO: 17; or
the first immunoglobulin single variable domain may comprise the CDR1, CDR2, and CDR3 of the VHH set forth in SEQ ID NO: 4, and the second immunoglobulin single variable domain may comprise the CDR1, CDR2, and CDR3 of the VHH set forth in SEQ ID NO: 20; or
the first immunoglobulin single variable domain may comprise the CDR1, CDR2, and CDR3 of the VHH set forth in SEQ ID NO: 9, and the second immunoglobulin single variable domain may comprise the CDR1, CDR2, and CDR3 of the VHH set forth in SEQ ID NO: 10; or
the first immunoglobulin single variable domain may comprise the CDR1, CDR2, and CDR3 of the VHH set forth in SEQ ID NO: 9, and the second immunoglobulin single variable domain may comprise the CDR1, CDR2, and CDR3 of the VHH set forth in SEQ ID NO: 14; or
the first immunoglobulin single variable domain may comprise the CDR1, CDR2, and CDR3 of the VHH set forth in SEQ ID NO: 9, and the second immunoglobulin single variable domain may comprise the CDR1, CDR2, and CDR3 of the VHH set forth in SEQ ID NO: 17; or
the first immunoglobulin single variable domain may comprise the CDR1, CDR2, and CDR3 of the VHH set forth in SEQ ID NO: 9, and the second immunoglobulin single variable domain may comprise the CDR1, CDR2, and CDR3 of the VHH set forth in SEQ ID NO: 20; or
the first immunoglobulin single variable domain may comprise the CDR1, CDR2, and CDR3 of the VHH set forth in SEQ ID NO: 10, and the second immunoglobulin single variable domain may comprise the CDR1, CDR2, and CDR3 of the VHH set forth in SEQ ID NO: 14; or
the first immunoglobulin single variable domain may comprise the CDR1, CDR2, and CDR3 of the VHH set forth in SEQ ID NO: 10, and the second immunoglobulin single variable domain may comprise the CDR1, CDR2, and CDR3 of the VHH set forth in SEQ ID NO: 17; or
the first immunoglobulin single variable domain may comprise the CDR1, CDR2, and CDR3 of the VHH set forth in SEQ ID NO: 10, and the second immunoglobulin single variable domain may comprise the CDR1, CDR2, and CDR3 of the VHH set forth in SEQ ID NO: 20; or
the first immunoglobulin single variable domain may comprise the CDR1, CDR2, and CDR3 of the VHH set forth in SEQ ID NO: 14, and the second immunoglobulin single variable domain may comprise the CDR1, CDR2, and CDR3 of the VHH set forth in SEQ ID NO: 17; or
the first immunoglobulin single variable domain may comprise the CDR1, CDR2, and CDR3 of the VHH set forth in SEQ ID NO: 14, and the second immunoglobulin single variable domain may comprise the CDR1, CDR2, and CDR3 of the VHH set forth in SEQ ID NO: 20; or
the first immunoglobulin single variable domain may comprise the CDR1, CDR2, and CDR3 of the VHH set forth in SEQ ID NO: 17, and the second immunoglobulin single variable domain may comprise the CDR1, CDR2, and CDR3 of the VHH set forth in SEQ ID NO: 20.

In the present application, the CDR1, CDR2, and CDR3 in the VHH set forth in SEQ ID NO: 1, 4, 9, 10, 14, 17, or 20 are shown in the following table:

| VHH sequence | CDR1-3 according to Kabat | CDR1-3 according to AbM | CDR1-3 according to Chothia | CDR1-3 according to IMGT |
|---|---|---|---|---|
| SEQ ID NO:1 | SEQ ID NO:24-26 | SEQ ID NO:27-29 | SEQ ID NO:30-32 | SEQ ID NO:33-35 |
| SEQ ID NO:4 | SEQ ID NO:60-62 | SEQ ID NO:63-65 | SEQ ID NO:66-68 | SEQ ID NO:69-71 |
| SEQ ID NO:9 | SEQ ID NO:120-122 | SEQ ID NO:123-125 | SEQ ID NO:126-128 | SEQ ID NO:129-131 |
| SEQ ID NO:10 | SEQ ID NO:132-134 | SEQ ID NO:135-137 | SEQ ID NO:138-140 | SEQ ID NO:141-143 |
| SEQ ID NO:14 | SEQ ID NO:180-182 | SEQ ID NO:183-185 | SEQ ID NO:186-188 | SEQ ID NO:189-191 |
| SEQ ID NO:17 | SEQ ID NO:216-218 | SEQ ID NO:219-221 | SEQ ID NO:222-224 | SEQ ID NO:225-227 |
| SEQ ID NO:20 | SEQ ID NO:252-254 | SEQ ID NO:255-257 | SEQ ID NO:258-260 | SEQ ID NO:261-263 |

In the present application, the FXI-binding protein may comprise a first immunoglobulin single variable domain and a second immunoglobulin single variable domain, wherein
the first immunoglobulin single variable domain may comprise the amino acid sequence set forth in one of SEQ ID NOs: 1 and 300-305, and the second immunoglobulin single variable domain may comprise the amino acid sequence set forth in one of SEQ ID NOs: 4 and 306-311; or
the first immunoglobulin single variable domain may comprise the amino acid sequence set forth in one of SEQ ID NOs: 1 and 300-305, and the second immunoglobulin single variable domain may comprise the amino acid sequence set forth in SEQ ID NO: 9; or
the first immunoglobulin single variable domain may comprise the amino acid sequence set forth in one of SEQ ID NOs: 1 and 300-305, and the second immunoglobulin single variable domain may comprise the amino acid sequence set forth in one of SEQ ID NOs: 10 and 312-317; or
the first immunoglobulin single variable domain may comprise the amino acid sequence set forth in one of SEQ ID NOs: 1 and 300-305, and the second immunoglobulin single variable domain may comprise the amino acid sequence set forth in one of SEQ ID NOs: 14 and 318-323; or
the first immunoglobulin single variable domain may comprise the amino acid sequence set forth in one of SEQ ID NOs: 1 and 300-305, and the second immunoglobulin single variable domain may comprise the amino acid sequence set forth in one of SEQ ID NOs: 17 and 324-329; or
the first immunoglobulin single variable domain may comprise the amino acid sequence set forth in one of SEQ ID NOs: 1 and 300-305, and the second immunoglobulin single variable domain may comprise the amino acid sequence set forth in one of SEQ ID NOs: 20 and 330-335; or
the first immunoglobulin single variable domain may comprise the amino acid sequence set forth in one of SEQ ID NOs: 4 and 306-311, and the second immunoglobulin single variable domain may comprise the amino acid sequence set forth in SEQ ID NO: 9; or
the first immunoglobulin single variable domain may comprise the amino acid sequence set forth in one of SEQ ID NOs: 4 and 306-311, and the second immunoglobulin single variable domain may comprise the amino acid sequence of the VHH set forth in one of SEQ ID NOs: 10 and 312-317; or
the first immunoglobulin single variable domain may comprise the amino acid sequence set forth in one of SEQ ID NOs: 4 and 306-311, and the second immunoglobulin single variable domain may comprise the amino acid sequence set forth in one of SEQ ID NOs: 14 and 318-323; or
the first immunoglobulin single variable domain may comprise the amino acid sequence set forth in one of SEQ ID NOs: 4 and 306-311, and the second immunoglobulin single variable domain may comprise the amino acid sequence set forth in one of SEQ ID NOs: 17 and 324-329; or
the first immunoglobulin single variable domain may comprise the amino acid sequence set forth in one of SEQ ID NOs: 4 and 306-311, and the second immunoglobulin single variable domain may comprise the amino acid sequence set forth in one of SEQ ID NOs: 20 and 330-335; or
the first immunoglobulin single variable domain may comprise the amino acid sequence set forth in SEQ ID NO: 9, and the second immunoglobulin single variable domain may comprise the amino acid sequence set forth in one of SEQ ID NOs: 10 and 312-317; or
the first immunoglobulin single variable domain may comprise the amino acid sequence set forth in SEQ ID NO: 9, and the second immunoglobulin single variable domain may comprise the amino acid sequence set forth in one of SEQ ID NOs: 14 and 318-323; or
the first immunoglobulin single variable domain may comprise the amino acid sequence set forth in SEQ ID NO: 9, and the second immunoglobulin single variable domain may comprise the amino acid sequence set forth in one of SEQ ID NOs: 17 and 324-329; or
the first immunoglobulin single variable domain may comprise the amino acid sequence set forth in SEQ ID NO: 9, and the second immunoglobulin single variable domain may comprise the amino acid sequence set forth in one of SEQ ID NOs: 20 and 330-335; or
the first immunoglobulin single variable domain may comprise the amino acid sequence set forth in one of SEQ ID NOs: 10 and 312-317, and the second immunoglobulin single variable domain may comprise the amino acid sequence set forth in one of SEQ ID NOs: 14 and 318-323; or
the first immunoglobulin single variable domain may comprise the amino acid sequence set forth in one of SEQ ID NOs: 10 and 312-317, and the second immunoglobulin single variable domain may comprise the amino acid sequence set forth in one of SEQ ID NOs: 17 and 324-329; or
the first immunoglobulin single variable domain may comprise the amino acid sequence set forth in one of SEQ ID NOs: 10 and 312-317, and the second immunoglobulin single variable domain may comprise the amino acid sequence set forth in one of SEQ ID NOs: 20 and 330-335; or
the first immunoglobulin single variable domain may comprise the amino acid sequence set forth in one of SEQ ID NOs: 14 and 318-323, and the second immunoglobulin single variable domain may comprise the amino acid sequence set forth in one of SEQ ID NOs: 17 and 324-329; or
the first immunoglobulin single variable domain may comprise the amino acid sequence set forth in one of SEQ ID NOs: 14 and 318-323, and the second immunoglobulin single variable domain may comprise the amino acid sequence set forth in one of SEQ ID NOs: 20 and 330-335; or
the first immunoglobulin single variable domain may comprise the amino acid sequence set forth in one of SEQ ID NOs: 17 and 324-329, and the second immunoglobulin single variable domain may comprise the amino acid sequence set forth in one of SEQ ID NOs: 20 and 330-335.

In the present application, the FXI-binding protein may have the CDR1-CDR3 of the VHH set forth in SEQ ID NO: 14. For example, the FXI-binding protein may have the CDR1 (SEQ ID NO: 183) of the VHH set forth in SEQ ID NO: 14. For example, the FXI-binding protein may have the CDR2 (SEQ ID NO: 181) of the VHH set forth in SEQ ID NO: 14. For example, the FXI-binding protein may have the CDR3 (SEQ ID NO: 182) of the VHH set forth in SEQ ID NO: 14.

For example, the FXI-binding protein may have the CDR1 set forth in SEQ ID NO: 183, the CDR2 set forth in SEQ ID NO: 181, and the CDR3 set forth in SEQ ID NO: 182.

For example, the FXI-binding protein may have the VHH set forth in SEQ ID NO: 349.

In the present application, the FXI-binding protein may have the CDR1-CDR3 of the VHH set forth in SEQ ID NO: 17. For example, the FXI-binding protein may have the CDR1 (SEQ ID NO: 219) of the VHH set forth in SEQ ID NO: 17. For example, the FXI-binding protein may have the CDR2 (SEQ ID NO: 217) of the VHH set forth in SEQ ID NO: 17. For example, the FXI-binding protein may have the CDR3 (SEQ ID NO: 218) of the VHH set forth in SEQ ID NO: 17.

For example, the FXI-binding protein may have the CDR1 set forth in SEQ ID NO: 219, the CDR2 set forth in SEQ ID NO: 217, and the CDR3 set forth in SEQ ID NO: 218.

For example, the FXI-binding protein may have the VHH set forth in SEQ ID NO: 350.

For example, the FXI-binding protein may have the CDR1 set forth in SEQ ID NO: 183, the CDR2 set forth in SEQ ID NO: 181, and the CDR3 set forth in SEQ ID NO: 182; and the FXI-binding protein may have the CDR1 set forth in SEQ ID NO: 219, the CDR2 set forth in SEQ ID NO: 217, and the CDR3 set forth in SEQ ID NO: 218.

For example, the FXI-binding protein may have the VHH set forth in SEQ ID NO:349, and the FXI-binding protein may have the VHH set forth in SEQ ID NO: 350.

For example, the FXI-binding protein may have the amino acid sequence set forth in SEQ ID NO: 344.

In the present application, the first immunoglobulin single variable domain is located at the N terminus of the second immunoglobulin single variable domain. In some other embodiments, the second immunoglobulin single variable domain is located at the N terminus of the first immunoglobulin single variable domain.

In the present application, the FXI-binding protein of the present application may further comprise, in addition to at least one immunoglobulin single variable domain capable of specifically binding to FXI, an immunoglobulin Fc region. The FXI-binding protein of the present application may comprise the immunoglobulin Fc region, so as to allow the binding molecule to form a dimer. The Fc regions useful in the present application may be from different subtypes of immunoglobulins, for example, IgG (e.g., IgG1, IgG2, IgG3, or IgG4 subtype), IgA1, IgA2, IgD, IgE, or IgM.

In the present application, mutations may be introduced into the wild-type Fc sequence for altering Fc-mediated related activities. Such mutations include, but are not limited to: a) a mutation that alters Fc-mediated CDC activity; b) a mutation that alters Fc-mediated ADCC activity; or c) a mutation that alters FcRn-mediated half-life in vivo. Such mutations are described in the following documents: Leonard G Presta, Current Opinion in Immunology 2008, 20:460-470; Esohe E. Idusogie et al., J Immunol 2000, 164:4178-4184; RAPHAEL A. CLYNES et al., Nature Medicine, 2000, Volume 6, Number 4:443-446; Paul R. Hinton et al., J Immunol, 2006, 176:346-356. For example, mutations on 1, 2, 3, 4, 5, 6, 7, 8, 9, or 10 amino acids of the CH2 region may be used to increase or remove Fc-mediated ADCC or CDC activity or to enhance or reduce FcRn affinity. In addition, the stability of the protein may be increased by mutating 1, 2, 3, 4, 5, 6, 7, 8, 9 or 10 amino acids of the hinge region.

In the present application, mutations may be introduced into the Fc sequence, thereby making the mutated Fc more susceptible to the formation of homodimers or heterodimers. Ridgway, Presta et al. 1996 and Carter 2001 mentioned the knob-hole model that utilizes the steric effect of amino acid side chain groups on the Fc contact interface, which makes it easier to form heterodimers between different Fc mutations; for example, in CN102558355A or CN103388013A, the ionic interaction force between the Fc contact interfaces is changed by changing the charges of the amino acids of the Fc contact interfaces, so that heterodimers (CN102558355A) are more easily formed between different Fc mutation pairs, or homodimers (CN103388013A) are more easily formed between Fc with the same mutation.

The immunoglobulin Fc region may be a human immunoglobulin Fc region, and may be an Fc region of human IgG1. In the present application, the amino acid sequence of the immunoglobulin Fc region is set forth in SEQ ID NO: 336.

In the present application, in the FXI-binding protein of the present application, the immunoglobulin Fc region (e.g., the Fc region of human IgG1) is linked to the C terminus of the immunoglobulin single variable domain (e.g., the VHH) directly or indirectly via a linker (e.g., a peptide linker).

In the present application, the FXI-binding protein of the present application may comprise an immunoglobulin single variable domain that specifically binds to FXI linked, directly or via a linker, to an immunoglobulin Fc region allowing the FXI-binding protein to form a dimeric molecule that may comprise two FXI-binding domains. Such an FXI-binding protein is also referred to as a bivalent FXI-binding protein. In the present application, the dimer is a homodimer.

In the present application, the FXI-binding protein of the present application may comprise two immunoglobulin single variable domains specifically binding to FXI and one immunoglobulin Fc region allowing the FXI-binding protein to form a dimeric molecule comprising four FXI-binding domains, the two immunoglobulin single variable domains and the immunoglobulin Fc region being linked to each other directly or via a linker. Such an FXI-binding protein is also referred to as a tetravalent FXI-binding protein. In the present application, the dimer is a homodimer. In the present application, the two immunoglobulin single variable domains in the FXI-binding protein that specifically bind to FXI bind to different regions or different epitopes of FXI, respectively.

In the present application, the FXI-binding protein of the present application is capable of inhibiting the activity of FXI. In the present application, the FXI-binding protein of the present application is capable of inhibiting the coagulation function of FXI.

### Nucleic acid, vector, and host cell

In another aspect, the present application relates to a nucleic acid molecule encoding the FXI-binding protein of the present application. The nucleic acid of the present application may be RNA, DNA, or cDNA. According to one embodiment of the present application, the nucleic acid of the present application is a substantially isolated nucleic acid.

The nucleic acid of the present application may also be in the form of a vector (such as a plasmid, cosmid, or YAC), may be present in a vector, and/or may be part of a vector. The vector may especially be an expression vector, i.e., a vector that may provide the expression of the FXI-binding protein *in vitro* and/or *in vivo* (i.e., in a suitable host cell, host organism, and/or expression system). The expression vector may generally comprise at least one nucleic acid of the present invention operably linked to one or more suitable expression regulatory elements (e.g., promoters, enhancers, and terminators). The selection of the elements and sequences thereof for expression in a particular host is common knowledge of those skilled in the art. Specific examples of regulatory elements and other elements useful or necessary for the expression of the FXI-binding protein of the present application include, for example, promoters, enhancers, terminators, integration factors, selection markers, leader sequences, and reporter genes.

The nucleic acid of the present application may be prepared or acquired by known means (e.g., by automated DNA synthesis and/or recombinant DNA techniques) based on information about the amino acid sequence of the polypeptide of the present application given herein, and/or may be isolated from a suitable natural source.

In another aspect, the present application relates to a recombinant host cell expressing or capable of expressing one or more FXI-binding proteins of the present application and/or comprising the nucleic acid or the vector of the present application. Feasible host cells of the present application may be a bacterial cell, a fungal cell, or a mammalian cell.

Suitable bacterial cells include cells of Gram-negative bacterial strains (e.g., *Escherichia coli* strains, *Proteus* strains, and *Pseudomonas* strains) and Gram-positive bacterial strains (e.g., *Bacillus* strains, *Streptomyces* strains, *Staphylococcus* strains, and *Lactococcus* strains).

Suitable fungal cells include cells of species of *Trichoderma, Neurospora,* and *Aspergillus;* or include cells of species of *Saccharomyces* (e.g., *Saccharomyces cerevisiae), Schizosaccharomyces* (e.g., *Schizosaccharomyces pombe*)*, Pichia* (e.g., *Pichia pastoris* and *Pichia methanolica*) and *Hansenula.*

Suitable mammalian cells include, for example, HEK293 cells, CHO cells, BHK cells, HeLa cells, COS cells, and the like.

However, amphibian cells, insect cells, plant cells, and any other cells used in the art for the expression of heterologous proteins may also be used in the present application.

The present application also provides a method for producing an FXI-binding protein of the present application, which generally comprises the following steps:
- culturing the host cell of the present application in a condition allowing the expression of the FXI-binding protein of the present application; and
- isolating the FXI-binding protein expressed by the host cell from the culture; and
- optionally further purifying and/or modifying the FXI-binding protein of the present application.

The FXI-binding protein of the present application may be produced intracellularly (e.g., in the cytoplasm, in the periplasm, or in inclusion bodies) in the cells as described above, followed by isolation from the host cell and optionally further purification; or it may be produced extracellularly (e.g., in a medium in which the host cell is cultured), followed by isolation from the medium and optionally further purification.

Methods and reagents for recombinant production of polypeptides, e.g., specifically suitable expression vectors, transformation or transfection methods, selection markers, methods of inducing protein expression, culture conditions, etc., are known in the art. Similarly, protein isolation and purification techniques suitable for use in the methods for producing the FXI-binding protein of the present application are well known to those skilled in the art.

However, the FXI-binding protein of the present application may also be obtained by other protein production methods known in the art, such as chemical synthesis, including solid-phase or liquid-phase synthesis.

### Pharmaceutical composition

In another aspect, the present application provides a composition, e.g., a pharmaceutical composition, comprising one or a combination of the FXI-binding proteins of the present application formulated together with a pharmaceutically acceptable carrier. Such compositions may comprise one or a combination (e.g., two or more different) of the FXI-binding proteins of the present application. For example, the pharmaceutical composition of the present application may comprise a combination of antibody molecules that bind to different epitopes on the target antigen (FXI).

As used herein, the "pharmaceutically acceptable carrier" includes any and all solvents, dispersion media, coatings, antibacterial and antifungal agents, isotonic and absorption delaying agents, and the like that are physiologically compatible. Feasibly, the carrier is suitable for intravenous, intramuscular, subcutaneous, parenteral, spinal, or epidermal administration (e.g., by injection or infusion). Depending on the route of administration, the active compound, i.e., the antibody molecule, may be encapsulated in a material to protect the compound from acids and other natural conditions that may inactivate the compound.

The pharmaceutical composition of the present application may also comprise a pharmaceutically acceptable antioxidant. Examples of pharmaceutically acceptable antioxidants include: (1) water-soluble antioxidants, such as ascorbic acid, cysteine hydrochloride, sodium bisulfate, sodium metabisulfite and sodium sulfite; (2) oil-soluble antioxidants, such as ascorbyl palmitate, butylated hydroxyanisole (BHA), butylated hydroxytoluene (BHT), lecithin, propyl gallate and α-tocopherol; and (3) metal chelating agents, such as citric acid, ethylenediamine tetraacetic acid (EDTA), sorbitol, tartaric acid and phosphoric acid.

These compositions may also comprise, for example, preservatives, wetting agents, emulsifying agents, and dispersing agents.

Prevention of the presence of microorganisms can be ensured by sterilization procedures or by the inclusion of various antibacterial and antifungal agents, such as *p*-hydroxylbenzoate, chlorobutanol, phenol and sorbic acid. In many cases, the composition may comprise isotonic agents, for example, sugars, and polyalcohols such as mannitol, sorbitol, or sodium oxide. Prolonged absorption of injectable drugs can be achieved by adding to the composition an absorption delaying agent such as monostearate salts and gelatin.

The pharmaceutically acceptable carrier includes sterile aqueous solutions or dispersions and powders for the extemporaneous preparation of sterile injectable solutions or dispersions. The use of such media and reagents for pharmaceutically active substances is well known in the art. Conventional media or reagents, except incompatible with the active compounds, may be in the pharmaceutical composition of the present application. Supplementary active compounds may also be incorporated into the composition.

Therapeutic compositions generally must be sterile and stable in preparation and storage conditions. The composition may be formulated as solutions, microemulsions, liposomes or other ordered structures suitable for high drug concentrations. The carrier may be a solvent or dispersing agent comprising, for example, water, ethanol, polyol (for example, glycerol, propylene glycol, and liquid polyethylene glycol), and suitable mixtures thereof. Proper fluidity can be maintained, for example, by the use of a coating, such as lecithin, by maintaining the required particle size in the case of dispersions and by the use of surfactants.

Sterile solutions for injection may be prepared by incorporating an active compound at a required amount into an appropriate solvent with one or a combination of ingredients enumerated above, as required, followed by sterile microfiltration. Generally, dispersions are prepared by incorporating an active compound into a sterile carrier which comprises a basic dispersion medium and the required other ingredients from those enumerated above. For sterile powders used for preparing sterile solutions for injection, feasible preparation methods are vacuum drying and freeze-drying (lyophilization) that yield a powder of the active ingredient and any additional required ingredient from a previously sterilized solution thereof.

The amount of active ingredient that can be combined with a carrier material to prepare a single dosage form will vary depending upon the subject of interest and the particular mode of administration. The amount of active ingredient that can be combined with a carrier material to prepare a single dosage form will generally be an amount of the composition that produces a therapeutic effect. Typically, such an amount ranges from about 0.01% to about 99%, for example from about 0.1% to about 70%, or from about 1% to about 30%, of the active ingredient, based on 100%, in combination with a pharmaceutically acceptable carrier.

Dosage regimens can be adjusted to provide the best desired response (e.g., therapeutic response). For example, a single bolus injection may be given, several divided doses may be administered over time, or the doses may be proportionally reduced or increased as required by the exigencies of the therapeutic situation. It is particularly advantageous to formulate a parenteral composition into a unit dose form for ease of administration and uniformity of doses. The unit dose form as used herein refers to a physically discrete unit suitable as a unit dose for the subject of interest; each unit contains a predetermined quantity of the active compound calculated to produce a required therapeutic effect in combination with a required pharmaceutical carrier. The specific description of the unit dose form of the present application is defined and directly dependent upon (a) the unique characteristics of the active compound and the particular therapeutic effect to be achieved, and (b) the limitations inherent in the art of formulating such active compounds for the treatment of sensitivity in individuals.

For administration of the antibody molecules, the dose range is about 0.0001 mg/kg to 100 mg/kg, more often 0.01 mg/kg to 30 mg/kg body weight of the subject. For example, the dose may be 0.3 mg/kg body weight, 1 mg/kg body weight, 3 mg/kg body weight, 5 mg/kg body weight, 10 mg/kg body weight, 20 mg/kg body weight, or 30 mg/kg body weight, or within a range of 1-30 mg/kg body weight. Exemplary treatment regimens require administration once every week, once every two weeks, once every three weeks, once every four weeks, once every month, once every 3 months, once every 3-6 months, or require a slightly shorter administration interval (e.g., once every week to once every three weeks) followed by longer post-administration intervals (e.g., once every month to once every 3-6 months).

Or, the antibody molecules may be administered as a sustained-release preparation, in which case less frequent administration is required. The dose and frequency will vary depending on the half-life of the antibody molecules in the patient. Typically, human antibodies exhibit the longest half-life, followed by humanized antibodies, chimeric antibodies, and non-human antibodies. The dose and frequency of administration will vary depending on whether the treatment is prophylactic or therapeutic. In prophylactic use, relatively lower doses are administered at a lower frequency over a longer period. Some patients will continue to receive the treatment for the rest of life. In therapeutic use, it is sometimes desirable to administer higher doses at shorter intervals until the progression of the disease is reduced or halted, or feasibly, until the patient exhibits partial or complete amelioration of disease symptoms. Thereafter, the administration to the patient may be conducted according to the prophylactic regimen.

Actual dose levels of the active ingredients in the pharmaceutical composition of the present application may be varied so as to give amounts of the active ingredients that are effective to achieve the required therapeutic response for a particular patient, composition, and mode of administration, without toxicity in the patient. The selected dose level will depend upon a variety of pharmacokinetic factors including the activity of the particular composition of the present application employed, or an ester, salt or amide thereof, the route of administration, the time of administration, the rate of excretion of the particular compound employed, the duration of treatment, other drugs, compounds and/or materials used in combination with the particular composition employed, the age, sex, body weight, condition, general health and medical history of the patient of interest, and like factors well known in the medical arts.

The composition of the present application may be administered via one or more routes of administration using one or more methods well known in the art. It will be appreciated by those skilled in the art that the route and/or mode of administration will vary depending on the desired result. Feasible routes of administration of the FXI-binding protein of the present application include intravenous, intramuscular, intradermal, intraperitoneal, subcutaneous, spinal, or other parenteral routes of administration, such as injection or infusion. The phrase "parenteral administration" as used herein refers to a mode of administration other than enteral and topical administrations, typically injection, including, but not limited to, intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion.

Alternatively, the FXI-binding protein of the present application may also be administered through non-parenteral routes, such as topical, epidermal, or mucosal routes, e.g., intranasal, oral, vaginal, rectal, sublingual, or topical administration.

Without being bound by any theory, the following examples are intended only to illustrate the fusion protein, preparation method, use, etc., of the present application, and are not intended to limit the scope of the present application.

### Examples

### Example 1. Preparation of coagulation factor XI (FXI)-binding proteins

KN060 is an anti-FXI bispecific single-domain antibody fusion protein selected from a camel immune bank. It is a homodimer formed by 2 identical peptide chains, wherein the amino acid sequence of the peptide chain is set forth in SEQ ID NO: 344.

The reference antibody 14E11 is an anti-FXI antibody developed by Aronora. The amino acid sequence of the light chain of 14E11 is set forth in SEQ ID NO: 346, and the amino acid sequence of the heavy chain is set forth in SEQ ID NO: 345.

The reference antibody BAY1213790 is an anti-FXI antibody developed by Bayer. The amino acid sequence of the light chain of BAY1213790 is set forth in SEQ ID NO: 348, and the amino acid sequence of the heavy chain is set forth in SEQ ID NO: 347.

Vectors containing the nucleic acid sequences encoding the fusion proteins were transfected into cells, and the fusion proteins were acquired by expression and purification.

### Example 2. Affinity assay of coagulation factor XI (FXI)-binding proteins for FXI

### 2.1 Affinity of KN060 for FXI

(1) The binding kinetics of KN060 to hFXI-CHis protein were measured by bio-layer interferometry (BLI) using a molecular interaction instrument. The specific procedures are as follows: KN060 was diluted to 10 µg/mL and immobilized on a Protein A biosensor. hFXI-Chis protein was diluted to 50 nM, 25 nM, 12.5 nM, 6.25 nM, and 3.125 nM and bound to KN060. The results were fitted by adopting a 1:1 model, and the equilibrium dissociation constant (KD) value of the KN060 for binding to hFXI-Chis protein was calculated. 4E11 and BAY1213790 were used as the references. The affinity of KN060 to hFXI-Chis protein was 6.74E-10 KD (M). The affinity of reference 14E11 for hFXI-Chis protein was 3.02E-09 (M). The affinity of reference BAY1213790 was 4.12E-09 (M). It can be seen that the affinity of KN060 for hFXI-CHis is slightly higher than those of the 2 references.

**Table 1. Affinity results for KN060 for human FXI**

| Sample | KD (M) |
|---|---|
| KN060 | 6.74E-10 |
| 14E11 | 3.02E-09 |
| BAY1213790 | 4.12E-09 |

(2) KN060 was diluted to a final concentration of 10 µg/mL and directly immobilized on an AHC biosensor. For the kinetic assay, hApple-Chis (Accession No. P03951 in Uniprot database; the Apple domain amino acid sequence containing the first 387 positions was selected) was diluted to 5 concentrations, and subjected to 60 s of baseline detection, 120 s of association, and 900 s of dissociation. The diluent was a kinetic buffer, the regeneration solution was glycine-HCl (pH 1.7), and the neutralization solution was the diluent. The biosensor was Protein A biosensor. The association rate (kon) and dissociation rate (kdis) were calculated using a simple one-to-one Languir binding model (Octet K2 data analysis software version 9.0 (Data analysis 9.0)). The equilibrium dissociation constant (KD) was calculated as the ratio of kdis/kon.

The measured affinity of KN060 for hApple-Chis protein was <1.0E-12 KD (M).

The above results indicate that KN060 exhibits good affinities for human FXI factor protein and human Apple domain.

### 2.2. Affinity of KN060 for FXIa

To investigate whether KN060 can bind to FXIa, the affinity of KN060 for human FXIa (hFXIa) was evaluated by bio-layer interferometry (BLI). KN060 was diluted to 10 µg/mL and immobilized on a Protein A biosensor. hFXIa was diluted to 400 nM, 200 nM, 100 nM, 50 nM, and 25 nM, and bound to KN060. The results were fitted by adopting a 1:1 model, and the equilibrium dissociation constant (KD) value of the KN060 for binding to human FXIa was calculated.

The results show that the KD value of KN060 binding to hFXIa was 1.88E-09 M.

**Table 2. Affinity results for KN060 for hFXIa**

| Sample | KD (M) | Ka (1/Ms) | Kd (1/s) |
|---|---|---|---|
| KN060 | 1.88E-09 | 8.01E+04 | 1.50E-04 |

### 2.3 Affinity of KN060 for cynomolgus and rabbit FXI

The affinity of KN060 for cynomolgus and rabbit FXI was evaluated by bio-layer interferometry (BLI). KN060 stock solution 201113DS was diluted to 10 µg/mL and immobilized on a Protein A biosensor. Cynomolgus and rabbit FXI-apple proteins were diluted to 50 nM, 25 nM, 12.5 nM, 6.25 nM, and 3.125 nM, and bound to KN060. The results were fitted by adopting a 1:1 model, and the equilibrium dissociation constant (KD) values of the samples were calculated.

The results show that KN060 binds to both cynomolgus and rabbit FXI with KD values of 1.520E-13 M and 1.834E-10 M, respectively, suggesting that KN060 has a higher affinity for cynomolgus FXI than for rabbit FXI.

**Table 3. Affinity results of KN060 for cynomolgus and rabbit FXI**

| Sample | KD (M) | Ka (1/Ms) | Kd (1/s) |
|---|---|---|---|
| cynoFXI-Apple-Chis | 1.520E-13 | 3.175E+05 | 4.825E-08 |
| rabFXI-Apple-Chis | 1.834E-10 | 3.170E+05 | 5.816E-05 |

### Example 3. Inhibitory effect assay of coagulation factor XI (FXI)-binding proteins on FXI activity

### 3.1 Inhibitory effect of KN060 on FXI activity in vitro

The inhibitory activity of KN060 on FXI in vitro was analyzed by measuring the effect of KN060 on APTT (activated partial thromboplastin time) of standard human plasma on an automatic coagulation analyzer. The KN060 sample was diluted to 9.766 ng/mL-20000 ng/mL by using a coagulation calibrator. APTT of samples treated with different concentrations of KN060 was determined, and the FXI activity values were calculated through a calibration curve of the coagulation calibrator. The data were processed by using SoftMax Pro software. A curve of measured mean FXI activity vs. KN060 concentration was plotted by 4-parameter fitting. The inhibitory effect of KN060 on FXI activity was calculated and analyzed according to the detection data and the incorporated function Relative Potency* 100% of the SoftMax Pro software.

The results show that KN060 has a significant inhibitory effect on FXI activity in vitro, and the IC₅₀ value of the inhibitory effect on FXI activity was 650 ng/mL.

**Table 4. Inhibitory activity results for KN060 of different concentrations on FXI**

| Concentration (ng/mL) | Mean FXI activity (%) |
|---|---|
| 20000.000 | 2 |
| 10000.000 | 2 |
| 5000.000 | 2 |
| 2500.000 | 2 |
| 1250.000 | 19 |
| 625.000 | 56 |
| 312.500 | 78 |
| 156.250 | 97 |
| 78.125 | 100 |
| 39.063 | 95 |
| 19.531 | 101 |
| 9.766 | 101 |
| IC₅₀ value (ng/mL) | 650 |

### Example 4. Prolongation effect or inhibitory effect of KN060 on plasma APTT

Anti-FXI antibodies may prolong plasma APTT (activated partial thromboplastin time) by binding to FXI. The study determined the prolongation effect of KN060 on APTT (including folds and period of prolongation) in the plasma of different species on an automatic coagulation analyzer. The coagulation analyzer analysis of APTT is a common assay for evaluating the intrinsic coagulation pathway. The process comprises the addition of proper amounts of phospholipid and a surface activator, and incubation of plasma to activate the intrinsic coagulation factors, and the addition of calcium ions to trigger the coagulation process.

### 4.1 Prolongation effect on human plasma APTT

In human plasma, KN060, 14E11, and BAY1213790 at concentrations of 10 µg/mL, 5 µg/mL, 2 µg/mL, 1 µg/mL, and 0.5 µg/mL have certain prolongation effects on APTT in standard human plasma, with concentration dependence. Compared with the blank human plasma, the maximum prolongation folds of KN060, 14E11, and BAY1213790 on APTT were 3.71, 2.09, and 2.22, respectively. The prolongation effect of KN060 on human plasma APTT was significantly superior to those of 14E11 and BAY1213790. The results are shown in FIG. 1 and Table 5.

**Table 5. Prolongation folds of KN060, 14E11, and BAY1213790 on APTT in standard human plasma**

| Sample | APTT ratio | | | | | |
|---|---|---|---|---|---|---|
| | 10 µg/mL | 5 µg/mL | 2 µg/mL | 1 µg/mL | 0.5 µg/mL | 0.1 µg/mL |
| KN060 | 3.71 | 3.53 | 2.19 | 1.30 | 1.12 | 1.00 |
| 14E11 | 2.09 | 2.05 | 1.67 | 1.23 | 1.09 | 1.00 |
| BAY1213790 | 2.22 | 1.98 | 1.58 | 1.27 | 1.14 | 1.00 |

### 4.2 Prolongation effect on cynomolgus plasma APTT

In cynomolgus plasma, KN060 and 14E11 at concentrations of 10 µg/mL, 5 µg/mL, 2 µg/mL, 1 µg/mL, and 0.5 µg/mL have certain prolongation effects on APTT in cynomolgus plasma, with concentration dependence. Compared with the blank plasma, the maximum prolongation folds of KN060 and 14E11 on APTT were 3.38 and 2.53, respectively. The prolongation effect of KN060 on cynomolgus plasma APTT was significantly superior to that of 14E11. The results are shown in FIG. 2 and Table 6.

**Table 6. Prolongation folds of KN060 and 14E11 on APTT in cynomolgus plasma**

| Sample | APTT ratio | | | | | |
|---|---|---|---|---|---|---|
| | 10 µg/mL | 5 µg/mL | 2 µg/mL | 1 µg/mL | 0.5 µg/mL | 0.1 µg/mL |
| KN060 | 3.38 | 3.30 | 2.73 | 1.36 | 1.13 | 1.00 |
| 14E11 | 2.53 | 2.46 | 1.89 | 1.18 | 1.07 | 1.00 |

### 4.3 Prolongation effect on rabbit plasma APTT

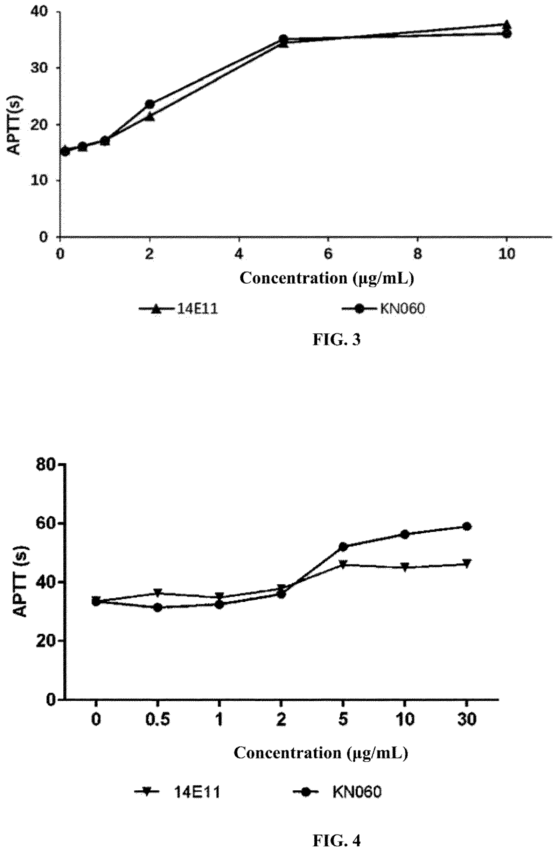

In rabbit plasma, KN060 and 14E11 at concentrations of 10 µg/mL, 5 µg/mL, and 2 µg/mL have certain prolongation effects on APTT in rabbit plasma, with concentration dependence. Compared with the blank plasma, the maximum prolongation folds of KN060 and 14E11 on APTT were 1.96 and 2.05, respectively. KN060 showed a similar prolongation effect on APTT in rabbit plasma to that of 14E11. The results are shown in FIG. 3 and Table 7.

**Table 7. Prolongation folds of KN060 and 14E11 on APTT in rabbit plasma**

| Sample | APTT ratio | | | | | |
|---|---|---|---|---|---|---|
| | 10 µg/mL | 5 µg/mL | 2 µg/mL | 1 µg/mL | 0.5 µg/mL | 0.1 µg/mL |
| KN060 | 1.96 | 1.91 | 1.28 | 0.93 | 0.88 | 0.83 |
| 14E11 | 2.05 | 1.88 | 1.17 | 0.93 | 0.88 | 0.85 |

### 4.4 Prolongation effect on rat plasma APTT

In rat plasma, KN060 and 14E11 at concentrations of 30 µg/mL, 10 µg/mL, and 5 µg/mL have certain prolongation effects on APTT in rat plasma, with concentration dependence. Compared with the blank plasma, the maximum prolongation folds of KN060 and 14E11 on APTT were 1.76 and 1.37, respectively. KN060 showed a similar prolongation effect on APTT in rat plasma to that of 14E11. The results are shown in FIG. 4 and Table 8.

**Table 8. Prolongation folds of KN060 and 14E11 on APTT in rat plasma**

| Sample | APTT ratio | | | | | |
|---|---|---|---|---|---|---|
| | 30 µg/mL | 10 µg/mL | 5 µg/mL | 2 µg/mL | 1 µg/mL | 0.5 µg/mL |
| KN060 | 1.76 | 1.67 | 1.55 | 1.07 | 0.97 | 0.94 |
| 14E11 | 1.37 | 1.34 | 1.37 | 1.13 | 1.04 | 1.08 |

### 4.5 Inhibitory activity against APTT in human whole plasma

The whole blood APTT was measured after the dilution of FXI antibodies to a variety of concentrations in standard human plasma (purchased from Sigma) and incubation at 37 °C for 3 minutes. 14E11 and BAY1213790 were used as the positive controls.

**Table 9**

| Concentration (µg/mL) | KN060 | BAY1213790 | 14E11 |
|---|---|---|---|
| 10 | 85.4 | 51.1 | 48.1 |
| 5 | 81.1 | 45.6 | 47.2 |
| 2 | 50.4 | 36.4 | 38.3 |
| 1 | 30.0 | 29.2 | 28.3 |
| 0.5 | 25.8 | 26.3 | 25.1 |
| 0.1 | 22.9 | 22.9 | 22.9 |

Table. 9 shows the variations of APTT with antibody concentration. The results show that KN060 can effectively prolong the whole blood APTT coagulation time, and exhibits a superior inhibitory effect against blood coagulation.

### 4.6 Inhibitory activity against APTT in monkey whole plasma

The whole blood APTT was measured after the dilution of KN060 to a variety of concentrations in monkey plasma (purchased from Sigma) and incubation at 37 °C for 3 minutes. 14E11 and BAY1213790 were used as the positive controls.

The results are shown in Table 10.

**Table 10**

| Concentration (µg/mL) | KN060 | BAY1213790 | 14E11 |
|---|---|---|---|
| 10 | 70.6 | 42.5 | 52.8 |
| 5 | 69.0 | 37.1 | 51.4 |
| 2 | 57.0 | 30.2 | 39.6 |

| | | | |
|---|---|---|---|
| 1 | 28.5 | 25.6 | 24.7 |
| 0.5 | 23.6 | 23.1 | 22.4 |
| 0.1 | 20.8 | 21.0 | 20.9 |

### 4.7 Inhibitory activity against APTT in rabbit whole plasma

The whole blood APTT was measured by diluting KN060 and standards to a variety of concentrations with rabbit plasma (purchased from Sigma). 14E11 and BAY1213790 were used as the positive controls.

The results are shown in Table 11.

**Table 11**

| Concentration (µg/mL) | KN060 | BAY1213790 | 14E11 |
|---|---|---|---|
| 10 | 36.1 | 28.8 | 37.8 |
| 5 | 35.1 | 24.1 | 34.5 |
| 2 | 23.6 | 18.1 | 21.5 |
| 1 | 17.1 | 16.4 | 17.2 |
| 0.5 | 16.2 | 16.0 | 16.1 |
| 0.1 | 15.3 | 15.3 | 15.6 |

The above results indicate that KN060 has superior APTT inhibitory activities in human or monkey plasma to the positive control antibodies.

### Example 5. In vivo efficacy study of KN060

(1) A pharmacodynamic study of thrombosis prevention was conducted by a single prophylactic administration of KN060 at different doses in a rabbit AV-shunt thrombus model.

A total of 24 male common New Zealand rabbits were randomized into 4 groups by body weight (G1 - vehicle control group; G2 - KN060, 1 mpk; G3 - KN060, 0.4 mpk; G4 - KN060, 0.04 mpk). The animals were kept in an anesthetic state. The left carotid artery and the right jugular vein were connected via a PE pipeline containing a 10-cm suture and pre-filled with normal saline. The animals were administered 15 min before the connection. After the connection, the animals were perfused for 30 min to allow thrombosis.

After the modeling was finished, the suture in the pipeline was taken out, and the blood was removed by a filter paper. The wet weight of the thrombus was weighed on a balance. The plasma was collected pre-dose, at the end of modeling, and 30 min, 60 min and 2 h after the end of modeling, and APTT and PT (prothrombin time) were determined. Meanwhile, at the end of modeling, the whole blood was collected, PRP (platelet-rich plasma) and PPP (platelet-poor plasma) were separated, and the ADP (adenosine diphosphate)-induced platelet aggregation was measured to evaluate the platelet aggregation capacity. In addition, at 5 min after the end of modeling, a bleeding test was conducted by measuring the bleeding amount and the bleeding time, so as to evaluate the effect of KN060 on hemostasis.

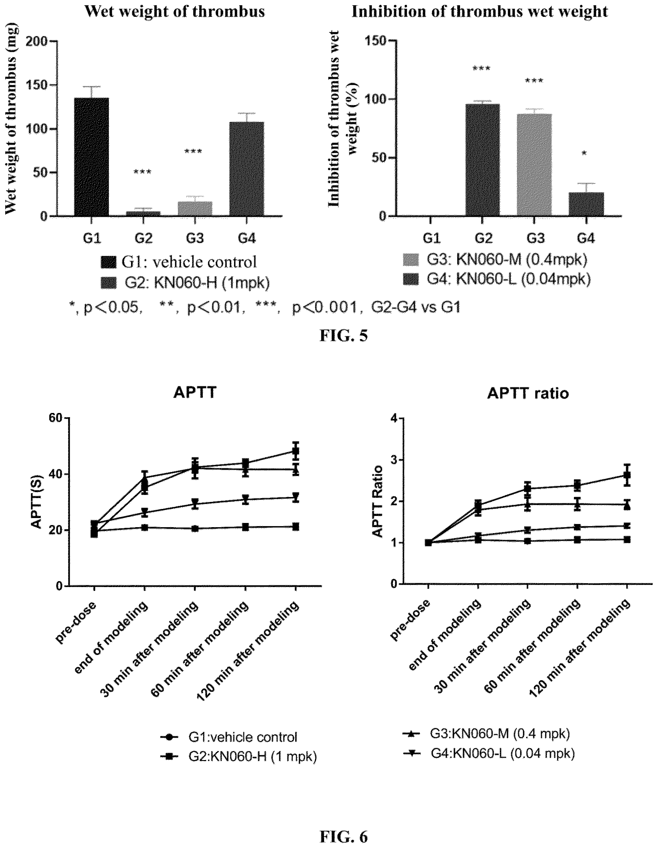

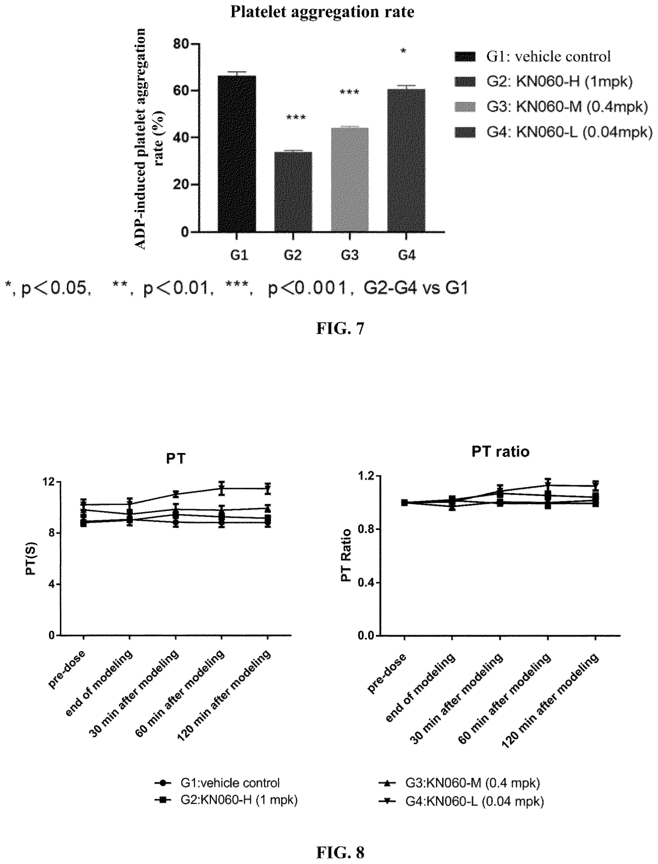

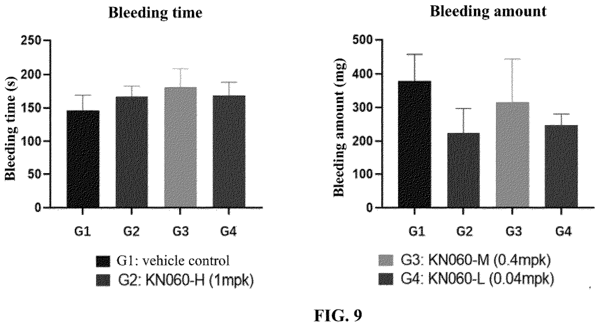

The results show that the model animals receiving 0.04, 0.4, and 1 mg/kg of KN060 intravenously exhibited significant effects of inhibiting the thrombus weight (see FIG. 5 and Table 12) and prolonging the APTT (see FIG. 6 and Table 14). The mean inhibition rates of thrombus wet weight in low-, medium-, and high-dose treatment groups were 20.57%, 87.55%, and 95.87%, respectively (see FIG. 5 and Table 13); at 120 min after the end of modeling, the APTTs of the low-, medium-, and high-dose treatment groups were prolonged by 1.41, 1.92, and 2.64 folds on average (see FIG. 6 and Table 15). The efficacy exhibits significant dose dependence. At the end of modeling, the ADP-induced platelet aggregation capacity was also determined, and the mean platelet aggregation rates of the vehicle control group and the KN060 low-, medium- and high-dose treatment groups were 66.29%, 60.59%, 44.08%, and 33.62%, respectively, demonstrating a negative correlation between platelet aggregation rate and dose (see FIG. 7 and Table 16). KN060 treatment had no significant effect on PT and bleeding (see FIGs. 8 and 9 and Tables 17, 18, 19, and 20).

In conclusion, in the rabbit AV-shunt thrombus model, different doses of KN060 can reduce the weight of thrombus, prolong APTT, and reduce the platelet aggregation rate dose-dependently, thus showing significant efficacy in preventing thrombus and no significant effect on PT and hemostasis.

**Table 12. Inhibitory effect of KN060 at different doses on thrombus weight**

| Wet weight of thrombus (mg) | | | | |
|---|---|---|---|---|
| | G1 | G2 | G3 | G4 |
| Mean | 135.63 | 5.60 | 16.88 | 107.73 |
| SEM | 12.55 | 3.59 | 5.80 | 10.25 |

**Table 13. Thrombus weight inhibition rate in KN060 treatment groups at different doses**

| Inhibition rate of thrombus wet weight (%) | | | | |
|---|---|---|---|---|
| | G1 | G2 | G3 | G4 |
| Mean | 0.00 | 95.87 | 87.55 | 20.57 |
| SEM | 0.00 | 2.64 | 4.28 | 7.56 |

**Table 14. Prolongation effect of KN060 at different doses on APTT**

| APTT (S) | | | | | | |
|---|---|---|---|---|---|---|
| Group | | Pre-dose | End of modeling | 30 min after modeling | 60 min after modeling | 120 min after modeling |
| G1 | Mean | 19.77 | 20.97 | 20.55 | 21.12 | 21.27 |
| | SEM | 0.44 | 0.72 | 0.63 | 1.05 | 1.04 |
| G2 | Mean | 18.58 | 35.22 | 42.47 | 44.00 | 48.33 |
| | SEM | 0.55 | 2.14 | 1.86 | 1.32 | 3.06 |
| G3 | Mean | 21.88 | 38.80 | 42.12 | 41.80 | 41.73 |
| | SEM | 1.03 | 2.19 | 3.57 | 2.47 | 1.89 |
| G4 | Mean | 22.45 | 26.23 | 29.35 | 30.90 | 31.68 |
| | SEM | 0.41 | 1.32 | 1.59 | 1.42 | 1.50 |

**Table 15. APTT prolongation folds in KN060 treatment groups at different doses**

| APTT ratio | | | | | | |
|---|---|---|---|---|---|---|
| Group | | Pre-dose | End of modeling | 30 min after modeling | 60 min after modeling | 120 min after modeling |
| G1 | Mean | 1.00 | 1.06 | 1.04 | 1.07 | 1.08 |
| | SEM | 0.00 | 0.05 | 0.05 | 0.06 | 0.06 |
| G2 | Mean | 1.00 | 1.90 | 2.30 | 2.38 | 2.64 |
| | SEM | 0.00 | 0.12 | 0.15 | 0.12 | 0.25 |
| G3 | Mean | 1.00 | 1.79 | 1.93 | 1.93 | 1.92 |
| | SEM | 0.00 | 0.13 | 0.15 | 0.14 | 0.11 |
| G4 | Mean | 1.00 | 1.17 | 1.31 | 1.38 | 1.41 |
| | SEM | 0.00 | 0.05 | 0.06 | 0.05 | 0.05 |

**Table 16. Platelet aggregation rate after treatment with different doses of KN060**

| Platelet aggregation rate (%) | | | | |
|---|---|---|---|---|
| | G1 | G2 | G3 | G4 |
| Mean | 66.29 | 33.62 | 44.08 | 60.59 |
| SEM | 1.83 | 0.74 | 0.66 | 1.67 |

**Table 17. Effect of KN060 at different doses on PT**

| PT(s) | | | | | | |
|---|---|---|---|---|---|---|
| Group | | Pre-dose | End of modeling | 30 min after modeling | 60 min after modeling | 120 min after modeling |
| G1 | Mean | 8.90 | 9.05 | 8.83 | 8.82 | 8.82 |
| | SEM | 0.36 | 0.46 | 0.35 | 0.37 | 0.33 |
| G2 | Mean | 8.82 | 9.00 | 9.43 | 9.27 | 9.15 |
| | SEM | 0.08 | 0.16 | 0.20 | 0.20 | 0.22 |
| G3 | Mean | 9.82 | 9.47 | 9.85 | 9.78 | 9.93 |
| | SEM | 0.40 | 0.21 | 0.42 | 0.34 | 0.25 |
| G4 | Mean | 10.22 | 10.25 | 11.03 | 11.48 | 11.47 |
| | SEM | 0.42 | 0.44 | 0.23 | 0.51 | 0.40 |

**Table 18. PT fold-changes in KN060 treatment groups at different doses**

| PT ratio | | | | | | |
|---|---|---|---|---|---|---|
| Group | | Pre-dose | End of modeling | 30 min after modeling | 60 min after modeling | 120 min after modeling |
| G1 | Mean | 1.00 | 1.02 | 0.99 | 0.99 | 0.99 |
| | SEM | 0.00 | 0.01 | 0.02 | 0.03 | 0.02 |
| G2 | Mean | 1.00 | 1.02 | 1.07 | 1.05 | 1.04 |
| | SEM | 0.00 | 0.03 | 0.02 | 0.03 | 0.03 |
| G3 | Mean | 1.00 | 0.97 | 1.00 | 1.00 | 1.02 |
| | SEM | 0.00 | 0.03 | 0.02 | 0.03 | 0.03 |
| G4 | Mean | 1.00 | 1.00 | 1.09 | 1.13 | 1.13 |
| | SEM | 0.00 | 0.03 | 0.04 | 0.05 | 0.03 |

**Table 19. Effect of KN060 at different doses on bleeding time**

| Bleeding time (s) | | | | |
|---|---|---|---|---|
| | G1 | G2 | G3 | G4 |
| Mean | 146.17 | 166.67 | 181.17 | 168.67 |
| SEM | 22.69 | 16.14 | 27.76 | 19.96 |

**Table 20. Effect of KN060 at different doses on bleeding amount**

| Bleeding amount (mg) | | | | |
|---|---|---|---|---|
| | G1 | G2 | G3 | G4 |
| Mean | 379.55 | 224.80 | 316.80 | 247.40 |
| SEM | 77.72 | 72.68 | 128.48 | 33.47 |

(2) The rabbits were fasted at night, and were anesthetized by injecting an anesthetic via the ear vein after blood was collected through the ear vein. The jugular vein was ligated at the distal end and the proximal end, and the interval between two ligatures was kept at about 3.0 cm. 15 min before molding, 1 mg/kg of the test sample or the PBS negative control was intravenously administered via the ear vein. Artery clamps were applied to the proximal end and the distal end of the jugular vein, and blood in the blood vessel was completely extracted from the facial vein by using a syringe. 0.3 mL of an agonist at 5 mg/mL was injected into the closed section of the blood vessel, and after 5 minutes of incubation, the agonist was drained using the syringe, followed by rinsing with normal saline 2 times. The artery clamps were removed to restore the blood flow, and the diameter of the blood vessel was controlled at 0.8 mm, so as to induce thrombosis. After the restoration of blood flow for 25 minutes, the distal end and the proximal end of the closed section of the vein were clamped by artery clamps. The ligatures at the proximal end and the distal end of the vein were tightened, the closed vein was cut, and the thrombus was taken out to determine and record the wet weight of the thrombus. After the thrombus was dried in a 60 °C oven for 20 h, the dry weight of the thrombus was measured and recorded.

The measurement was thrombus weight, and the experimental data were represented by X±SD. The significance test was performed using GraphPad Prism 5 one-way ANOVA (see Table. 21).

**Table 21**

| Group | Thrombus weight (mg/cm) | | | | | | | | | |
|---|---|---|---|---|---|---|---|---|---|---|
| PBS | 4.2333 | 55.88333 | 30.88952 | 46.2 | 5.529167 | 5.933333 | 25.21667 | 94.11812 | 25.21667 | 29.78885 |
| 14E11 1mg/kg | 30.71429 | 47.43227 | 55.80357 | 25.49769 | 32.01417 | 2530662 | | | | |
| B1213790-F11a 1mg/kg | 11.03333 | 21.85714 | 73.99537 | 0 | | | | | | |
| KN060 1mg/kg | 63.17778 | 191.9768 | 15.02167 | 21.39968 | | | | | | |

### Example 6. Specificity of KN060

KN060 obtained in the above examples was selected and the non-specific binding thereof to other coagulation-related proteins was examined by bio-layer interferometry (BLI). The antibody proteins were immobilized onto AHC chips and the proteins examined were commercially available FVII, FIX, FV, FXII, pro-thrombin, α-kallikrein, FVIIa, FIXa, FVa, FXIIa, and Thrombin. The experimental results show that all bispecific antibodies do not bind to FVII, FIX, FV, FXII, pro-thrombin, α-kallikrein, FVIIa, FIXa, FVa, FXIIa, and Thrombin.

### Example 7. Clinical study of KN060

### 1. Safety and tolerability assessment of a single KN060 intravenous drip in healthy subj ects

Study protocol:
Study design: A randomized, double-blind, placebo-controlled phase I clinical study evaluating the safety, tolerability, pharmacokinetics, and pharmacodynamics of KN060 injections in Chinese healthy subjects
Number of subjects: 38 healthy subjects were planned.

The specific grouping and mode of administration are as follows:
Treatment groups: a single intravenous drip of KN060 injection in 6 dose groups of 0.1 mg/kg, 0.3 mg/kg, 1.0 mg/kg, 2.5 mg/kg, 5.0 mg/kg and 10.0 mg/kg;
Control group: a single intravenous drip of placebo.

Subjects meeting all of the following criteria could be enrolled: 1, healthy male or postmenopausal/amenorrheic female; 2, aged between 18 and 55 years (inclusive) when providing the informed consent; 3, body mass index (BMI) between 19.0 and 26.0 kg/m² (exclusive); male ≥ 50.0 kg, and female ≥ 45.0 kg; 4, normal activated partial thrombin time (APTT), prothrombin time (PT), international normalized ratio (INR), and platelet count; 5, capable of understanding the procedures of the study protocol, and willing to accept and comply with the requirements of the protocol.

Endpoints:
1) Adverse events (D1-D56);
2) Plasma concentration and PK relevant parameters (D1-D56);
3) APTT, FXI activity, total FXI, and free FXI results (D1-D56);
4) Immunogenicity (D1-D56).

Preliminary results show that KN060 has good safety, pharmacokinetics, and pharmacodynamics in humans.

### 2. Efficacy and safety assessment of a single KN060 intravenous drip in patients with elective total knee arthroplasty

Study protocol:
Study design: a randomized, open-label, positive-control, multi-center study comparing the efficacy and safety of different doses of KN060 and enoxaparin in preventing venous thromboembolism in patients with elective unilateral knee arthroplasty Number and grouping of subjects: about 400 subjects were planned and would be randomized into KN060 low- and high-dose treatment groups and an LMWH group (low-molecular-weight heparin)
Mode of administration: single intravenous drip
Time of administration: 6 to 8 h after the operation (not more than 24 h after incision closure)

Major inclusion and exclusion criteria
1) Inclusion criteria: male or female (female without child-bearing potential), aged between 18 and 75 years (inclusive); planned selective unilateral TKA surgery
2) Exclusion criteria: hemorrhagic disease or high risk of hemorrhage; VTE history or ongoing uninterruptible anticoagulation therapy; severe hepatic and renal insufficiency; contraindication of angiography; active malignant tumors under treatment; requiring regional anesthesia, such as spinal or epidural anesthesia; requiring epidural analgesia before or after the surgery.

Primary endpoints
1) Primary efficacy endpoints: incidence of venous thromboembolism (including venographically confirmed DVT, symptomatic DVT and PE, fatal PE, and death of unknown cause where PE cannot be excluded) on days 10-14 post-surgery;
2) Primary safety endpoint: incidence of clinically relevant bleeding (defined as major bleeding or clinically relevant non-major bleeding) from signing the informed consent form (ICF) to day 14 post-surgery;
3) PK&PD endpoints (secondary): some subjects might be subjected to PK & PD tests and correlation analyses of efficacy and safety endpoints.

The foregoing detailed description is provided by way of illustration and example and is not intended to limit the scope of the appended claims. Various variants of the embodiments exemplified in the present application will be apparent to those of ordinary skills in the art, and shall fall within the scope of the appended claims and equivalents thereof.

## Claims

1. A method for preventing and/or treating a thromboembolic disease, comprising:
administering to a subject in need a coagulation factor XI (FXI)-binding protein comprising a first immunoglobulin single variable domain and a second immunoglobulin single variable domain, wherein the first immunoglobulin single variable domain comprises the CDR1, CDR2,
and CDR3 of the VHH set forth in SEQ ID NO: 14, and the second immunoglobulin single variable domain comprises the CDR1, CDR2, and CDR3 of the VHH set forth in SEQ ID NO: 17.

2. The method according to claim 1, wherein the thromboembolic disease includes venous thromboembolism (VTE).

3. The method according to claim 2, wherein the venous thromboembolism is a complication of tumor and/or a complication of arthroplasty.

4. The method according to claim 2 or 3, wherein the venous thromboembolism is associated with a peripherally inserted central catheter (PICC).

5. The method according to any one of claims 1-4, wherein the subject has, or is at risk of having, venous thromboembolism (VTE).

6. The method according to any one of claims 1-5, wherein the subject is a tumor patient.

7. The method according to claim 6, wherein the subject has a peripherally inserted central catheter (PICC).

8. The method according to any one of claims 2-7, wherein the venous thromboembolism is associated with hip and/or knee arthroplasty.

9. The method according to any one of claims 1-8, wherein the subject has received arthroplasty.

10. The method according to claim 9, wherein the hip and/or knee joint of the subject has been replaced.

11. The method according to any one of claims 1-10, wherein the thromboembolic disease includes deep vein thrombosis (DVT).

12. The method according to claim 11, wherein the deep vein thrombosis includes acute deep vein thrombosis and/or recurrence of deep vein thrombosis after acute deep vein thrombosis.

13. The method according to any one of claims 2-12, wherein the venous thromboembolism includes pulmonary thromboembolism (PTE).

14. The method according to any one of claims 1-13, wherein the subject has, or is at risk of having, deep vein thrombosis (DVT).

15. The method according to any one of claims 1-14, wherein the subject has acute deep vein thrombosis and/or recurrence of deep vein thrombosis after acute deep vein thrombosis.

16. The method according to any one of claims 1-15, wherein the subject has pulmonary thromboembolism (PTE).

17. The method according to any one of claims 1-16, wherein the thromboembolic disease includes systemic thromboembolism.

18. The method according to claim 17, wherein the systemic thromboembolism is a complication of tumor, a complication of atrial fibrillation, and/or a complication of dialysis.

19. The method according to claim 18, wherein the atrial fibrillation includes non-valvular atrial fibrillation.

20. The method according to any one of claims 1-19, wherein the subject has, or is at risk of having, systemic thromboembolism.

21. The method according to any one of claims 1-20, wherein the subject has non-valvular atrial fibrillation.

22. The method according to any one of claims 1-21, wherein the subject is a patient receiving dialysis.

23. The method according to any one of claims 1-22, wherein the subject is an adult.

24. The method according to any one of claims 1-23, wherein the subject has hypertension, diabetes, congestive heart failure, atrial fibrillation, and/or a history of stroke.

25. The method according to any one of claims 23-24, wherein the subject is aged at least 75 years.

26. The method according to any one of claims 1-25, wherein the subject is bedridden.

27. The method according to any one of claims 1-26, wherein the CDRs are Kabat CDRs, AbM CDRs, Chothia CDRs, or IMGT CDRs.

28. The method according to any one of claims 1-27, wherein the CDR1, CDR2, and CDR3 of the VHH set forth in SEQ ID NO: 14 are selected from any one of the following groups: SEQ ID NOs: 180-182, SEQ ID NOs: 183-185, SEQ ID NOs: 186-188, and SEQ ID NOs: 189-191.

29. The method according to any one of claims 1-28, wherein the first immunoglobulin single variable domain comprises the amino acid sequence set forth in one of SEQ ID NOs: 14 and 318-323.

30. The method according to any one of claims 1-29, wherein the CDR1, CDR2, and CDR3 of the VHH set forth in SEQ ID NO: 17 are selected from any one of the following groups: SEQ ID NOs: 216-218, SEQ ID NOs: 219-221, SEQ ID NOs: 222-224, and SEQ ID NOs: 225-227.

31. The method according to any one of claims 1-30, wherein the second immunoglobulin single variable domain comprises the amino acid sequence set forth in one of SEQ ID NOs: 17 and 324-329.

32. The method according to any one of claims 1-31, wherein the coagulation factor XI (FXI)-binding protein further comprises an immunoglobulin Fc region.

33. The method according to any one of claims 1-32, wherein the coagulation factor XI (FXI)-binding protein is administered at a dose of about 0.5 mg/kg to about 10 mg/kg.

34. The method according to any one of claims 1-33, further comprising: administering to the subject: a nucleic acid molecule encoding the coagulation factor XI (FXI)-binding protein according to any one of claims 1-33, an expression vector that may comprise the nucleic acid molecule operably linked to an expression regulatory element, a cell that may comprise the nucleic acid molecule or may be transformed with the expression vector and is capable of expressing the coagulation factor XI (FXI)-binding protein, and/or a pharmaceutical composition that may comprise the coagulation factor XI (FXI)-binding protein and a pharmaceutically acceptable carrier.

35. Use of a coagulation factor XI (FXI)-binding protein in preparing a medicament for preventing and/or treating a thromboembolic disease, wherein the FXI-binding protein comprises a first immunoglobulin single variable domain comprising the CDR1, CDR2, and CDR3 of the VHH set forth in SEQ ID NO: 14, and a second immunoglobulin single variable domain comprising the CDR1, CDR2, and CDR3 of the VHH set forth in SEQ ID NO: 17.

36. A coagulation factor XI (FXI)-binding protein for use in preventing and/or treating a thromboembolic disease, wherein the FXI-binding protein comprises a first immunoglobulin single variable domain comprising the CDR1, CDR2, and CDR3 of the VHH set forth in SEQ ID NO: 14, and a second immunoglobulin single variable domain comprising the CDR1, CDR2, and CDR3 of the VHH set forth in SEQ ID NO: 17.
